# FASCICULE DE BREVET EUROPEEN

(11) **EP 2 087 134 B1**
(45) Date de publication et mention de la délivrance du brevet: **10.08.2011**
(21) Numéro de dépôt: 07866534.6
(22) Date de dépôt: 20.11.2007
(51) Int. Cl.: C12Q 1/68

(54) **METHODE DE DIAGNOSTIC ET DE SUIVI D'UNE VAGINOSE BACTERIENNE PAR QUANTIFICATION MOLECULAIRE**
VERFAHREN ZUR DIAGNOSE UND VERFOLGUNG VON BAKTERIELLER VAGINOSE MITTELS MOLEKULARER QUANTIFIZIERUNG
METHOD FOR DIAGNOSIS OF AND FOLLOWING A BACTERIAL VAGINOSIS BY MOLECULAR QUANTIFICATION

(30) Priorité: 24.11.2006 FR 0610313
(43) Date de publication de la demande: 12.08.2009
(73) Titulaire: Université de la Méditerranée (Aix-Marseille II), 13284 Marseille Cédex 07 (FR)
(72) Inventeur: BRETELLE, Florence, 13014 Marseille (FR); FENOLLAR, Florence, 13008 Marseille (FR); HENRY-MARY, Mireille, 13850 Greasque (FR); MENARD, Jean-Pierre, 13008 Marseille (FR); RAOULT, Didier, 13008 Marseille (FR)
(74) Mandataire: Domange, Maxime
(86) Numéro de dépôt international: PCT/FR2007/052368
(87) Numéro de publication internationale: WO 2008/062136

(56) Documents cités:
- US-A1- 2005 095 603
- BRADSHAW C S ET AL: "The association of Atopobium vaginae and Gardnerella vaginalis with bacterial vaginosis and recurrence after oral metronidazole therapy" JOURNAL OF INFECTIOUS DISEASES, CHICAGO, IL, US, vol. 194, no. 6, 15 septembre 2006 (2006-09-15), pages 828-836, XP009086643 ISSN: 0022-1899
- TABRIZI SEPEHR N ET AL: "Prevalence of Gardnerella vaginalis and Atopobium vaginae in virginal women" SEXUALLY TRANSMITTED DISEASES, LIPPINCOTT CO., PHILADELPHIA, US, vol. 33, no. 11, 1 novembre 2006 (2006-11-01), pages 663-665, XP009086642 ISSN: 0148-5717
- ZARIFFARD M REZA ET AL: "Detection of bacterial vaginosis-related organisms by real-time PCR for Lactobacilli, Gardnerella vaginalis and Mycoplasma hominis." 13 décembre 2002 (2002-12-13), FEMS IMMUNOLOGY AND MEDICAL MICROBIOLOGY 13 DEC 2002, VOL. 34, NR. 4, PAGE(S) 277 - 281 , XP002442546 ISSN: 0928-8244 page 278, colonne 1, alinéa 2 page 278, colonne 2, alinéas 2,3 tableau 1 le document en entier
- VERHELST RITA ET AL: "Comparison between Gram stain and culture for the characterization of vaginal microflora: Definition of a distinct grade that resembles grade I microflora and revised categorization of grade I microflora" BMC MICROBIOLOGY, BIOMED CENTRAL, LONDON, GB, vol. 5, no. 1, 14 octobre 2005 (2005-10-14), page 61, XP021002663 ISSN: 1471-2180
- VERHELST RITA ET AL: "Cloning of 16S rRNA genes amplified from normal and disturbed vaginal microflora suggests a strong association between Atopobium vaginae, Gardnerella vaginalis and bacterial vaginosis" 21 avril 2004 (2004-04-21), BMC MICROBIOLOGY, BIOMED CENTRAL, LONDON, GB, PAGE(S) 16 , XP021002564 ISSN: 1471-2180 abrégé tableau 1
- SCHWIERTZ ANDREAS ET AL: "Throwing the dice for the diagnosis of vaginal complaints?" ANNALS OF CLINICAL MICROBIOLOGY AND ANTIMICROBIALS, BIOMED CENTRAL, LPNDON, GB, vol. 5, no. 1, 17 février 2006 (2006-02-17), page 4, XP021018181 ISSN: 1476-0711
- FERRIS MICHAEL J ET AL: "Use of species-directed 16S rRNA gene PCR primers for detection of Atopobium vaginae in patients with bacterial vaginosis." décembre 2004 (2004-12), JOURNAL OF CLINICAL MICROBIOLOGY DEC 2004, VOL. 42, NR. 12, PAGE(S) 5892 - 5894 , XP002442547 ISSN: 0095-1137 le document en entier

### SEQUENCE LISTING

<110> UNIVERSITE DE LA MEDITERRANEE (Aix-Marseille II)
<120> Méthode de diagnostic et de suivi d'une vaginose bactérienne par quantification moléculaire
<130> H52 437 cas 19 FR
<160> 28
<170> PatentIn version 3.1
<210> 1
   <211> 135
   <212> DNA
   <213> Albumine humaine
<400> 1
   gctgtcatct cttgtgggct gtaatcatcg tttaagagta atattgcaaa acctgtcatg      60
   cccacacaaa tctctccctg gcattgttgt ctttgcagat gtcagtgaaa gagaaccagc     120
   agctcccatg agttt                                                       135
<210> 2
   <211> 81
   <212> DNA
   <213> atopobium vaginae
<400> 2
   ccctatccgc tcctgatacc ggcaggcttg agtctggtag gggaagatgg aattccaagt 60
   gtgaaatgcg cagatatttg g 81
<210> 3
   <211> 86
   <212> DNA
   <213> Gardnerella vaginalis
<400> 3
   cgcatctgct aaggatgttg aaacatcgtt taaggctact gcaactattt ctgcagcaga 60
   tcctgaagtt ggcgaaaaga ttgctg 86
<210> 4
   <211> 90
   <212> DNA
   <213> Lactobacillus sp.
<400> 4
   tacatcccaa ctccagaacg tgatactgac aagccattct taatgcagtt gaagacgtat 60
   ttactatcac tggtcgtggt actgttgctt 90
<210> 5
   <211> 20
   <212> DNA
   <213> atopobium vaginae
<400> 5
   ccctatccgc tcctgatacc 20
<210> 6
   <211> 20
   <212> DNA
   <213> atopobium vaginae
<400> 6
   ccaaatatct gcgcatttca 20
<210> 7
   <211> 23
   <212> DNA
   <213> atopobium vaginae
<400> 7
   gcaggcttga gtctggtagg gga 23
<210> 8
   <211> 20
   <212> DNA
   <213> Gardnerella vaginalis
<400> 8
   cgcatctgct aaggatgttg 20
<210> 9
   <211> 20
   <212> DNA
   <213> Gardnerella vaginalis
<400> 9
   cagcaatctt ttcgccaact 20
<210> 10
   <211> 24
   <212> DNA
   <213> Gardnerella vaginalis
<400> 10
   tgcaactatt tctgcagcag atcc 24
<210> 11
   <211> 20
   <212> DNA
   <213> Lactobacillus sp.
<400> 11
   tacatcccaa ctccagaacg 20
<210> 12
   <211> 20
   <212> DNA
   <213> Lactobacillus sp.
<400> 12
   aagcaacagt accacgacca 20
<210> 13
   <211> 21
   <212> DNA
   <213> Lactobacillus sp.
<400> 13
   tgacaagcca ttcttaatgc a 21
<210> 14
   <211> 22
   <212> DNA
   <213> Albumine humaine
<400> 14
   gctgtcatct cttgtgggct gt 22
<210> 15
   <211> 23
   <212> DNA
   <213> Albumine humaine
<400> 15
   aaactcatgg gagctgctgg ttc 23
<210> 16
   <211> 26
   <212> DNA
   <213> Albumine humaine
<400> 16
   cctgtcatgc ccacacaaat ctctcc 26
<210> 17
   <211> 141
   <212> DNA
   <213> artificial sequence
<220>
   <223> Fragment de l'exon 12 du gène de l'albumine humaine des positions 16283 à 16423 modifié par insertion du site de clivage XhoI
<400> 17
   gctgtcatct cttgtgggct gtaatcatcg tctcgagtta agagtaatat tgcaaaacct     60
   gtcatgccca cacaaatctc tccctggcat tgttgtcttt gcagatgtca gtgaaagaga    120
   accagcagct cccatgagtt t                                               141
<210> 18
   <211> 939
   <212> DNA
   <213> artificial sequence
<220>
   <223> Séquence combinant des séquences de Mobilincus curtisii, Mobilinc us mulieris, Gardnerella vaginalis, Lactobacillus sp, Ureaplasma urealyticum, Mycoplasma hominis, Atopobium vaginae, Candida albic ans et albumine humaine
<400> 18
   gccatggaaa aggtgggtca agagggcgtc atcaccgtgg aagaacatcg tctcgagtta     60
   aggagcctcg aagtcaccga aggtatgcgt ttcattatgg atatgcgtgt ggatggatta    120
   ctcgagctgc ctgttttggg tgggggcgct atcggggttt gggcttacat gcctggccct    180
   cgcatctgct aaggatgttg aaacatcgtc tcgagttaag gctactgcaa ctatttctgc    240
   agcagatcct gaagttggcg aaaagattgc tgaatacatc ccaactccag aacgtgatac    300
   tgacaagcca ttcttaatgc cagttgaaga cgtatttact atcactggtc gtggtactgt    360
   tgcttctata ctggtgaccg tcctatccaa gttggatcac atcgtctcga gttaagaaca    420
   aatagtgcat tagtattctt tgatgaaaaa ggaaacgaag acaaagaacg taaagttgct    480
   tatggacgtc gtttcgatat tccatcaggt aattgattgc tgcaggtgat acatttagag    540
   cagcggcagt tgaacaattc atcgtctcga gttaaggagt tggggctgat attgtaacac    600
   caaaccccta tccgctcctg ataccggcag gcttgagtct ggtaggggaa gatggaattc 660
   caagtctcga ggtgaaatgc gcagatattt ggaagccaac gccaacgaag acaaggcact 720
   tcaaaaagcc gatggtagta gaaaactgcg acatcgtctc gagttaagtt ggttgatgca 780
   aacaaagctg gtagttgctg tcatctcttg tgggctgtaa tcatcgtctc gagttaagag 840
   taatattgca aaacctgtca tgcccacaca aatctctccc tggcattgtt gtctttgcag 900
   atgtcagtga aagagaacca gcagctccca tgagtttgg 939
<210> 19
   <211> 178
   <212> DNA
   <213> artificial sequence
<220>
   <223> Séquence de Mobilincus curtisii et Mobilincus mulieris
<400> 19
   gccatggaaa aggtgggtca agagggcgtc atcaccgtgg aagaacatcg tctcgagtta 60
   aggagcctcg aagtcaccga aggtatgcgt ttcattatgg atatgcgtgt ggatggatta 120
   ctcgagctgc ctgttttggg tgggggcgct atcggggttt gggcttacat gcctggcc 178
<210> 20
   <211> 183
   <212> DNA
   <213> artificial sequence
<220>
   <223> Séquence de Gardnerella vaginalis et Lactobacillus sp
<400> 20
   cgaccagtga tagtaaatac gtcttcaact ggcattaaga atggcttgtc agtatcacgt 60
   tctggagttg ggatgtattc agcaatcttt tcgccaactt caggatctgc tgcagaaata 120
   gttgcagtag ccttaactcg agacgatgtt tcaacatcct tagcagatgc gagggccagg 180
   cat 183
<210> 21
   <211> 145
   <212> DNA
   <213> Ureaplasma urealyticum
<400> 21
   tcactggtcg tggtactgtt gcttctatac tggtgaccgt cctatccaag ttggatcaca 60
   tcgtctcgag ttaagaacaa atagtgcatt agtattcttt gatgaaaaag gaaacgaaga 120
   caaagaacgt aaagttgctt atgga 145
<210> 22
   <211> 188
   <212> DNA
   <213> Mycoplasma hominis
<400> 22
   cttggaattc catcttcccc taccagactc aagcctgccg gtatcaggag cggatagggg 60
   tttggtgtta caatatcagc cccaactcct taactcgaga cgatgaattg ttcaactgcc 120
   gctgctctaa atgtatcacc tgcagcaatc aattacctga tggaatatcg aaacgacgtc 180
   cataagca 188
<210> 23
   <211> 150
   <212> DNA
   <213> artificial sequence
<220>
   <223> Séquence de Atopobium vaginae et Candida albicans
<400> 23
   gaattccaag tctcgaggtg aaatgcgcag atatttggaa gccaacgcca acgaagacaa 60
   ggcacttcaa aaagccgatg gtagtagaaa actgcgacat cgtctcgagt taagttggtt 120
   gatgcaaaca aagctggtag ttgctgtcat 150
<210> 24
   <211> 145
   <212> DNA
   <213> Albumine humaine
<400> 24
   ccaaactcat gggagctgct ggttctcttt cactgacatc tgcaaagaca acaatgccag 60
   ggagagattt gtgtgggcat gacaggtttt gcaatattac tcttaactcg agacgatgat 120
   tacagcccac aagagatgac agcaa 145
<210> 25
   <211> 19
   <212> DNA
   <213> artificial sequence
<220>
   <223> amorce
<400> 25
   gccatggaaa aggtgggtc 19
<210> 26
   <211> 21
   <212> DNA
   <213> artificial sequence
<220>
   <223> amorce
<400> 26
   cctgatggaa tatcgaaacg a 21
<210> 27
   <211> 20
   <212> DNA
   <213> artificial sequence
<220>
   <223> amorce
<400> 27
   actggtgacc gtcctatcca 20
<210> 28
   <211> 20
   <212> DNA
   <213> artificial sequence
<220>
   <223> amorce
<400> 28
   ccaaactcat gggagctgct 20

## Description

La présente invention concerne une méthode de diagnostic et de suivi *in vitro* de l'état de la flore bactérienne vaginale au regard de la présence d'une vaginose bactérienne (VB) ou de son évolution, le cas échéant pour le suivi de sa prise en charge thérapeutique.

Les techniques actuelles de diagnostic de la VB, qui est une infection fréquente avec des conséquences néfastes pour la grossesse, reposent sur des critères qui sont peu fiables. Certaines bactéries ont été décrites comme étant associées à cette maladie, sans jamais avoir bénéficiées d'une qualification et d'une quantification moléculaire fiable.

Pendant longtemps, la VB a été définie du point de vue microbiologique par une quasi-disparition de la flore vaginale normale composée principalement de lactobacilles au profit d'autres bactéries, notamment *Gardnerella vaginalis, Mobiluncus* spp. et mycoplasmes génitaux [Spiegel CA, CMR 1991 ; Thorsen P, AJGO 1998]. La VB est un motif fréquent de consultation médicale, elle est notamment impliquée dans la susceptibilité aux infections sexuellement transmissibles comme le VIH, et pour les grossesses dans la prématurité et la naissance d'enfants de petits poids. Sa prévalence chez la femme, y compris durant la grossesse, se situe entre 8 et 23% [Guise JM, AJPM 2001] selon les méthodes actuelles d'investigation.

Cependant, la revue de la littérature est divergente quant à l'impact de la prise en charge thérapeutique de cette pathologie. En effet, les premières études, qui avaient mis en évidence une réduction du risque d'accouchement prématuré lors du traitement de la VB, n'ont pu être confirmées [Morales HJ, AJOG 1994 ; Hauth JC, NEJM 1995 ; Guise JM, AJPM 2001 ; McDonald H, CDSR 2005 ; Varma R, EJOGRB 2006 ; Okun N, OG, 2005 ; Leitich H, AJOG 2003 ; Guerra B, EJOGRB 2006].

L'objectif initial de la présente invention était d'évaluer l'impact de la VB pendant la grossesse ainsi que l'efficacité de la prise en charge thérapeutique de la VB pendant la grossesse. Mais, pour ce faire aucun outil diagnostique objectif n'était disponible. En effet, l'étude de la littérature révèle une grande confusion concernant la prise en charge thérapeutique de la VB liée principalement à l'absence d'outil rationnel pour le diagnostic et le suivi de cette pathologie.

Actuellement, les deux outils diagnostiques disponibles sont le score de Nugent et les critères d'Amsel. Le score de Nugent est la méthode la plus couramment rapportée dans la littérature, considérée par certains comme la technique de référence, même si elle n'est pas réalisée en routine dans les laboratoires de microbiologie clinique du fait du caractère fastidieux de sa mise en oeuvre [Fredricks DN, NEJM 2005 ; Thomason JL, AJOG 1992 ; Ison CA, STD 2002 ; Nugent RP, JCM 1991]. Le score de Nugent identifie la VB grâce à une analyse morphologique semi-quantitative des bactéries après coloration de Gram. C'est donc une technique subjective dont la reproductibilité a été mise en cause [Sha BE, JCM 2005 ; Schwebke JR, OG 1996]. Les critères cliniques d'Amsel (pH vaginal supérieur à 4,5 ; leucorrhées grisâtres homogènes adhérentes; odeur azotée après adjonction de KOH à 10%; présence de clue-cells) représentent la seconde approche diagnostique [Amsel R AJM 1983]. Comme le score de Nugent, il est de détermination délicate et non utilisé en pratique clinique courante.

Une des limites les plus péjoratives de ces méthodes diagnostiques est l'absence d'identification de certains microorganismes impliqués dans la VB. D'une part, l'absence de paroi des mycoplasmes ne permet pas leurs identifications par la coloration de Gram et donc leurs cotations par le score de Nugent. D'autre part, l'apport de la biologie moléculaire a permis d'identifier de nouvelles bactéries pouvant être impliquées dans la VB mais leur mise en évidence par les 2 méthodes diagnostiques existantes est impossible. *Atopobium vaginae* est la principale nouvelle espèce bactérienne caractérisée. Sa présence a été corrélée à la VB dans quelques articles sans toutefois qu'une appréciation quantitative fiable de sa place relative par rapport aux autres microorganismes ait été réalisée [Bradshaw CS, JID 2006, Rodriguez JM, IJSB 1999 ; Ferris MJ, BMCID 2004 ; Ferris MJ, JCM 2004 ; Verhelst R, BMCM 2004].

Dans l'article publié récemment par Bradshaw et col. [Bradshaw CS, JID 2006], il est notamment décrit une relation entre la détection des bactéries *A. vaginae* et *G. vaginalis* et la VB, mais ces résultats sont insuffisants pour réaliser un diagnostic de VB et/ou un suivi de l'évolution d'une VB fiables. En effet, les données présentées dans ce travail permettent le dépistage desdites bactéries et non pas leur réelle quantification. De plus, ce dépistage a montré une bonne sensibilité, *A. vaginae* et *G. vaginalis* étant détectées respectivement chez 96% et 99% des patientes atteintes de VB. Cependant, sa spécificité est mauvaise car *A. vaginae* est détectée chez 12% des patientes présentant une flore normale et *G. vaginalis* chez 60%.

Les auteurs ont alors tenté une approche dite « semi-quantitative » en classant les charges bactériennes comme faibles ou élevées par comparaison des CT (« Cycle Treshold ») médians de détection des microorganismes au sein de tous les échantillons analysés. Ainsi, les auteurs ont estimé une charge médiane de 4 x 10⁵ copies pour *G. vaginalis* (médiane correspondant à 21 cycles) et 4 x 10⁶ copies pour *A. vaginae* (médiane à 18 cycles). Les charges élevées de *G. vaginalis* (>4 x 10⁵) et d'*A. vaginae* (>4 x 10⁶) étaient significativement plus présentes chez les patientes présentant une VB par rapport aux patientes avec une flore normale. Cependant, ces valeurs présentaient une mauvaise sensibilité, *A. vaginae* et *G. vagina*/*is* étant seulement détectées respectivement chez 49% et 71% des patientes atteintes d'une VB (Table 1). De plus, 16 patientes (28%) avec une rechute de VB après traitement présentaient une concentration en *G. vaginalis* en dessous du seuil donné (Table 3). Quarante patientes (70%) avec une rechute de VB présentaient aussi une concentration en dessous du seuil en *A. vaginae.*

L'approche « semi-quantitative » des auteurs n'est donc pas applicable en tant qu'outil diagnostique et de suivi immédiat des patients. En fait, les techniques utilisées pour obtenir ces résultats étaient insuffisantes sur plusieurs points. Tout d'abord, les techniques de PCR n'étaient pas suffisamment sensibles car les cibles moléculaires amplifiaient de trop longs fragments (fragment de l'ARN 16S ribosomique de 430 paires de base pour *A. vaginae* et de 291 paires de base pour *G. vaginalis*). Il est actuellement établi qu'avec une séquence ciblée lors d'une réaction de PCR aussi longue, la sensibilité est faible. En plus, les techniques de PCR en temps réel utilisaient pour la détection et quantification un marquage en SybrGreen du produit d'amplification, méthode beaucoup moins spécifique que celles qui utilisent des sondes d'hydrolyse marquées qui nécessitent une triple spécificité (deux amorces plus la sonde, pour amplifier un fragment dont la taille n'excède pas 120 paires de bases). Les quantifications s'effectuent vraisemblablement avec un standard variable et non pas en fonction d'une gamme plasmidique stable, reproductible et comparable au cours du temps. Enfin, ils ne bénéficiaient pas d'un outil de contrôle quantitatif permettant de juger de la qualité de leurs prélèvements. En effet, il recherchait seulement la présence de β-globine humaine dans les échantillons sans la quantifier. Il est donc très difficile de comparer quantitativement les échantillons entre eux car la variation de la quantité des bactéries peut être liée à une variation qualitative et quantitative des sécrétions vaginales prélevées.

L'évaluation de la prise en charge thérapeutique des VB, ne peut donc se passer de la mise au point préalable d'un outil fiable adapté au diagnostic de la VB et au suivi qualitatif et quantitatif de la flore vaginale.

Le but de la présente invention est de fournir une méthode de diagnostic et de suivi de la VB à la fois plus fiable, plus précise et plus simple à mettre en oeuvre en routine dans les laboratoires d'analyse microbiologique clinique.

Pour ce faire, les inventeurs ont étudié les prélèvements vaginaux de 204 femmes enceintes, et recherché la présence de chaque micro-organisme impliqué dans la VB, en développant une méthode de PCR en temps réel permettant la détection de l'ADN spécifique des bactéries et la détermination de la charge bactérienne au moyen d'une gamme plasmidique établie grâce à la construction d'un plasmide étalon. Ce plasmide comprend les fragments d'ADN spécifiques desdites bactéries à amplifier et à quantifier et du gène de l'albumine humaine utilisé comme contrôle de la qualité de l'échantillon d'ADN et de l'amplification moléculaire ainsi que de la richesse de l'échantillon biologique. Les microorganismes cibles étudiés *(Lactobacillus sp*., *G. vaginalis, Mobilincus curtisii, Mobilincus Mulieris*, *Ureaplasma urealyticum, Mycoplasma bominis, A. vaginae,* et *Candida albicans*) étaient ceux décrits comme pouvant être impliqués dans la VB et/ou la prématurité. Les résultats de la quantification des divers microorganismes obtenus par biologie moléculaire ont été confrontés à la classification par le score de Nugent.

Selon la présente invention il a été démontré que la présence d'*A.vaginae* à partir d'un certain seuil de concentration peut être associée à une VB de façon très spécifique et significativement et que cette détection moléculaire rend le diagnostic facile et fiable.

L'existence d'un seuil de concentration spécifique de la VB pour la bactérie *G. vaginalis* a aussi été démontrée selon la présente invention.

En outre, il a été démontré que l'association des 2 bactéries à certaines concentrations permet le diagnostic de VB avec notamment une prédictivité positive supérieure à 95% et surtout une prédictivité négative supérieure à 99%.

Par ailleurs, au cours des VB, les bactéries du genre *Lactobacillus sp.,* normalement présentes dans le vagin, présentent une concentration qui va en diminuant et il a été démontré selon la présente invention qu'en deçà d'un certain seuil de concentration la quantification de ces bactéries *Lactobacillus sp.* permet de diagnostiquer de façon fiable une VB. Enfin, il a été démontré qu'une évolution du rapport des concentrations de *Lactobacillus sp.* vs l'addition des concentrations de *A. vaginae* et *G. vaginalis* permet d'évaluer l'évolution de la VB de façon fiable.

Plus précisément la présente invention fournit une méthode de diagnostic et suivi *in vitro* de l'état de la flore bactérienne vaginale au regard de la présence d'une vaginose bactérienne, et le cas échéant pour le suivi de son traitement thérapeutique, caractérisée en ce que
- 1 / on quantifie les concentrations de bactéries *Atopobium vaginae* et *Gardnerella vagina*/*is* en
   - déterminant les concentrations de séquences spécifiques desdites bactéries *Atopobium vaginae* et *Gardnerella vaginalis* présentes en une seule copie dans l'ADN desdites bactéries *Atopobium vaginae* et respectivement *Gardnerella vaginalis,* et d'une séquence spécifique d'un gène humain présent dans tout prélèvement biologique contenant des cellules humaines, dans l'ADN extrait d'un échantillon de sécrétion vaginale de patiente, lesdites séquences spécifiques présentant une taille inférieure à 150 nucléotides,
   - par co-amplification enzymatique de type PCR desdites séquences spécifiques contenues, d'une part, dans ledit ADN extrait de l'échantillon et, d'autre part, dans des échantillons de fragments d'ADN synthétiques comprenant chacun desdites séquences spécifiques desdites bactéries et dite séquence spécifique d'un gène humain présent dans tout prélèvement biologique de cellules humaines, lesdits échantillons servant de standards d'étalonnage de quantification de l'ADN,
   - les détection et quantification desdits fragments amplifiés étant réalisées à l'aide de sondes marquées de séquences distinctes de celles des amorces d'amplification pour chacune desdites séquences spécifiques desdites bactéries *Atopobium vaginae* et *Cardnerella vaginalis* et dite séquence spécifique d'un gène humain présent dans tout prélèvement biologique contenant des cellules humaines, et
   - lesdites séquences spécifiques desdites bactéries étant les séquences suivantes incluant des séquences desdites sondes (soulignées) encadrées par des séquences desdites amorces (en gras) ou leurs séquences complémentaires :
      - pour *Atopobium vaginae:*
      - pour *Gardnerella vaginalis:*
   et
- 2/ on détermine la présence d'une vaginose bactérienne ou l'échec du traitement thérapeutique en cours si les concentrations de fragments d'ADN des deux dites séquences spécifiques des bactéries *Atopobium vaginae* et respectivement *Gardnerella vaginalis* dans un échantillon de prélèvement de sécrétions vaginales de patiente contenant au moins 10⁴ cellules humaines/ml, sont telles que l'une au moins des 2 conditions a) et b) suivantes est respectée :
   a) la concentration Ca en dit fragment d'ADN d'*Atopobium vaginae* est supérieure ou égale à 10⁸ copies/ml, et
   b) la concentration Cg en dit fragment d'ADN de *Gardnerella vaginalis* est supérieure ou égale à 10⁹ copies/ml.

Une concentration de bactérie *Atopobium vaginae* supérieure ou égale au seuil de 108 permet de détecter environ 90% des vaginoses. La quantification de la bactérie *Gardnerella vaginalis* vient en complément dans le cas où la concentration en *Atopobium vaginae* serait inférieure au seuil de 10⁸ bactéries/ml, pour détecter une vaginose, car le seuil de détection de G. vaginalis supérieur ou égal à 10⁹ bactéries/ml ne permettrait à lui seul de détecter qu'environ la moitié des vaginoses. C'est pourquoi, selon la présente invention, il est nécessaire de quantifier les concentrations d'ADN pour les deux bactéries.

D'autre part, on relève que le développement d'une vaginose bactérienne est confirmé si les concentrations Ca, Cg et Cl d'au moins trois fragments de séquences spécifiques présents en une seule copie dans l'ADN des bactéries *A. vaginae* (Ca), *G. viginalis* (Cg) et respectivement *Lactobacillus sp.* (Cl) dans l'ADN extrait d'un échantillon de sécrétion vaginale de patiente sont telles que le rapport des concentrations Cl/(Ca+Cg) diminue entre les 2 échantillons prélevés successivement dans le temps à intervalle de temps suffisant, de préférence au moins 1 mois.

On entend ici par « développement d'une vaginose » une aggravation d'une vaginose déjà détectée ou, dans certains cas, un risque d'apparition d'une vaginose, c'est-à-dire d'un déséquilibre ou une anomalie de la flore vaginale risquant de devenir pathologique.

De même, l'échec du traitement en cours en fonction des concentrations des séquences spécifiques présentes en une seule copie dans l'ADN des bactéries est confirmé si le rapport des concentrations Cl/(Ca+Cg) diminue ou n'augmente pas entre les 2 échantillons prélevés à intervalle de temps suffisant, de préférence au moins 1 mois.

Plus particulièrement, on réalise une méthode dans laquelle :
- a/ à l'étape 1 /, on quantifie en outre les bactéries *Lactobacillus sp.* comprenant au moins les bactéries *Lactobacillus acidophillus, Lactobacillus crispatus, Lactobacillus jenseneii, Lactobacillus gasseri* et *Lactobacillus iners,*
   - en déterminant en outre la concentration d'une séquence spécifique desdites bactéries *Lactobacillus sp*., ladite séquence spécifique des *Lactobacillus* sp. étant présente en une seule copie dans l'ADN desdites bactéries Lactobacillus sp., et de taille inférieure à 150 nucléotides,
   - par co-amplification enzymatique de type PCR additionnelle de ladite séquence spécifique des bactéries Lactobacillus sp. contenue, d'une part, dans ledit ADN extrait de l'échantillon et, d'autre part, dans un échantillon de fragments d'ADN synthétique comprenant, en outre, ladite séquence spécifique desdites bactérie *Lactobacillus sp*., ledit fragment d'ADN synthétique comprenant ladite séquence spécifique desdites bactéries *Lactobacillus sp*. servant de standard d'étalonnage de quantification,
   - les détection et quantification desdits fragments amplifiés étant réalisées à l'aide de sondes marquées de séquences distinctes de celles des amorces d'amplification pour chacune desdites séquences spécifiques desdites bactéries *Atopobium vaginae*, *Gardnerella vaginalis*, *Lactobacillus sp*. et dite séquence spécifique d'un gène humain présent dans tout prélèvement biologique contenant des cellules humaines, et
- b/ à l'étape 2/, on détermine une vaginose bactérienne si, en outre, la concentration Cl en fragment d'ADN spécifique de dites bactéries *Lactobacillus sp*., est inférieure ou égale à 10⁸ copies/ml, de préférence inférieure ou égale à 10⁷ copies/ml.

Selon la présente invention, la concentration en bactéries du genre *Lactobacillus sp.* n'est pas suffisante pour conclure à la présence d'une vaginose bactérienne, mais vient en complément ou confirmation dans le cas où les conditions de concentrations en *A. vaginae* et *G. vaginalis* sont réunies.

De préférence, on détermine une vaginose bactérienne si lesdites concentrations sont telles que les 3 conditions suivantes sont respectées :
a- concentration Ca en dit fragment d'ADN de séquence spécifique d'*Atopobium vaginae* supérieure ou égale à 10⁸ copies/ml,
b- concentration Cg en en dit fragment d'ADN de séquence spécifique de *Gardnerella vaginalis* supérieure ou égale à 10⁹ copies/ml, et
c- concentration Cl en dit fragment d'ADN de séquence spécifique de *Lactobacillus sp.* inférieure ou égale à 10⁷ copies/ml.

Avantageusement, lesdites concentrations Ca, Cg ou Cl sont déterminées par amplification enzymatique de type PCR en temps réel et quantification de l'ADN desdits fragments d'ADN de séquences spécifiques des bactéries respectivement *Atopobium vaginae, Gardnerella vaginalis* et le cas échéant *Lactobacillus sp,* ainsi que, de préférence, d'un fragment d'ADN humain présent dans tout prélèvement biologique humain contenant des cellules.

De préférence, lesdites séquences spécifiques respectivement desdites bactéries *Atopobium vaginae, Gardnerella vaginalis,* et le cas échéant *Lactobacillus sp.* présentent une taille de 70 à 150 nucléotides de préférence de 90 à 120 nucléotides.

De préférence encore, on réalise des réactions d'amplification et quantification par PCR en Temps Réel, en mettant en oeuvre des sondes d'hydrolyse spécifiques respectivement de chacune desdites séquences spécifiques de dites bactéries et séquence spécifique d'un gène humain présent dans tout prélèvement biologique contenant des cellules humaines, dans l'échantillon à tester.

La technique de PCR en temps réel, consiste en une PCR classique en utilisant des amorces de séquence directe et inverse, et comprend une détection du produit amplifié basée sur la mesure d'émission de fluorescence proportionnelle à la quantité de gènes amplifiés avec une sonde dite « d'hydrolyse ». Pour cela, ladite sonde est marquée par un émetteur de fluorescence ou fluorophore en 5' et un agent bloquant l'émission de fluorescence en 3'. Cet agent bloquant absorbe la fluorescence émise lorsque le fluorophore et l'agent bloquant sont proches. Lorsque le fluorophore et l'agent bloquant sont séparés, l'émission de fluorescence n'est plus absorbée par l'agent bloquant. Lors de son passage, la Taq polymérase entraîne une hydrolyse de la sonde et donc une libération des nucléotides et du fluorophore en solution. L'émission de fluorescence sera donc proportionnelle au nombre d'amplifiat. Le principe de la PCR en temps réel repose sur la capacité de la Taq polymérase pendant l'étape d'élongation d'hydrolyser une sonde hybridée sur l'ADN à copier, cette hydrolyse permettant l'émission de fluorescence, laquelle permet une quantification. Au cours de la même réaction, on peut quantifier deux cibles différentes, en introduisant dans le mélange réactionnel deux amorces et une sonde dirigées contre une première cible, et deux autres amorces et sonde dirigées contre l'autre cible. Les deux sondes étant marquées avec des fluorophores différents.

De préférence encore, on met en oeuvre un grand fragment synthétique d'ADN servant de standard d'étalonnage de quantification de l'ADN, ledit grand fragment d'ADN synthétique regroupant desdites séquences spécifiques respectivement de chacune desdites bactéries dont les concentrations sont quantifiées, et ladite séquence d'ADN humain spécifique de cellules humaines. La présence de plusieurs cibles moléculaires sur un même fragment nucléique permet de quantifier des cibles différentes dans un même échantillon et de les coquantifier de façon homogène, la quantification en utilisant la même gamme d'étalonnage de plusieurs espèces moléculaires, permettant la comparaison de l'efficacité des différentes réactions de PCR entre elles et d'une détermination à l'autre au cours du temps et évite les biais liés au témoin positif.

On entend par "séquence spécifique de ladite bactérie", une séquence du génome de ladite bactérie qu'on ne retrouve dans aucun autre génome d'organisme vivant.

On entend par "fragment d'ADN", un fragment d'ADN ou oligonucléotide dont les séquences sont écrites ci-après dans le sens 5'→3'.

Plus particulièrement, lesdites séquences spécifiques desdites bactéries comprennent :
- pour la bactérie *Atopobium vaginae*: le fragment des positions 248 à 334 du gène ARN 16S ribosomal de référence Genebank AY 738658.1,
- pour la bactérie *Gardnerella vaginalis,* le fragment des positions 981 à 1072 du gène *Cpn 60* de la protéine chaperonne de 60kDa de référence Genebank AF 240579.3, et
- pour la bactérie *Lactobacillus sp.,* une séquence commune aux bactéries *Lactobacillus crispatus, Lactobacillus jenseneii*, *Lactobacillus gasseri*, *Lactobacillus ineri* et *Lactobacillus acidophilus* au sein du gène *tuf* codant pour le facteur d'élongation aux positions 253 à 343 du gène de référence Genebank AY 562191.1.

Plus particulièrement encore, lesdites séquences spécifiques desdites bactéries sont les séquences suivantes incluant des séquences sondes (soulignées) encadrées par des séquences amorces (en gras) ou leurs séquences reverses et complémentaires pour les amorces anti-sens:
- pour *Atopobium vaginae:*
- pour *Gardneralla vaginalis*:
- pour *Lactobacillus sp*.

Avantageusement, on réalise en outre la quantification des cellules humaines présentes dans ledit échantillon, notamment dans l'ADN obtenu à partir de prélèvement vaginal à tester, à titre de contrôle de la richesse du prélèvement, de la qualité de l'extraction de l'ADN, et de la présence potentielle d'inhibiteurs de la réaction de PCR. Pour ce faire, on quantifie le nombre de copies d'un gène humain présent dans tout prélèvement biologique humain contenant des cellules, notamment le nombre de copies du gène d'albumine humaine. La quantification du gène de l'albumine humaine sert ainsi de contrôle interne pour attester de la qualité et de la richesse de l'échantillon. De plus, au cours du suivi des patientes, cette quantification est un moyen de normalisation entre deux échantillons prélevés à des temps différents. En effet, le calcul du nombre de microorganismes par millions de cellules humaines autorise une comparaison inter-échantillon rigoureuse. Le gène de l'albumine est présent uniquement en 2 copies dans les cellules humaines et la mesure du signal de cette séquence permet donc la quantification du nombre de cellules humaines initiales dans le prélèvement. Une quantité de cellules inférieure ou égale à 50 pour 5 µl d'échantillon (10⁴ cellules/ml), soit une quantité d'ADN d'albumine inférieure ou égale à 10²/5 µl, amène à rejeter le prélèvement du fait d'une quantité insuffisante.

Du fait de la variabilité de qualité du prélèvement, du transport et de la conservation, la présente invention contient ainsi un témoin moléculaire de qualité permettant une systématisation du diagnostic avec une quantification fiable.

La quantification des cellules permet également de détecter l'absence des limiteurs de la réaction de PCR. En effet, lorsque l'échantillon est testé par PCR à différentes dilutions, la quantité d'albumine détectée augmente quand on augmente les dilutions en présence des limiteurs, alors qu'elle diminue quand on augmente les dilutions en l'absence des limiteurs de réaction PCR.

Plus particulièrement, on réalise en outre la quantification de l'ADN humain contenu dans ledit échantillon, et ledit grand fragment d'ADN comprend en outre une séquence spécifique d'ADN humain dans l'échantillon à tester telle qu'une séquence spécifique de l'albumine.

Plus particulièrement encore, ladite séquence spécifique d'ADN humain dans l'échantillon à tester comprend le fragment des positions 16283-16423 de l'exon 12 du gène de l'albumine humaine de référence Genebank M12523.1 de séquence du listage de séquence suivante ou la séquence complémentaire:

Seq. n°1= 5'-**GCTGTCATCTCTTGTGGGCTGT**AATCATCGTTTA AGAGTAATATTGCAAAACCTGTCATGCCCACACAAATCTCTCCCTGG CATTGTTGTCTTTGCAGATGTCAGTGAAAGA**GAACCAGCAGCTCCC ATGAGTTT**-3', de préférence une séquence Seq. n°1 modifiée par insertion d'un site de clivage, notamment le site XhoI (séquence rayée entre parenthèses) en dehors des séquences correspondant aux amorces (séquences en gras) et séquence sonde (séquence soulignée) comme la séquence Seq. n°17.

Avantageusement encore, on réalise des réactions d'amplification et de quantification en mettant en oeuvre des jeux d'amorces et des sondes d'hydrolyse spécifiques de chacune des différentes bactéries dites séquences spécifiques de chacune desdites bactéries à tester, et le cas échéant d'une séquence spécifique d'ADN humain dans l'échantillon à tester, telle que une séquence spécifique de l'albumine humaine, et ladite séquence spécifique comprend une séquence sonde encadrée par des séquences aptes à servir comme amorce dans une réaction d'amplification du type PCR desdites séquences spécifiques.

On entend ici par "sonde", un oligonucléotide, de préférence encore de 20 à 30 nucléotides, s'hybridant spécifiquement avec ladite séquence spécifique et donc permettant de la détecter et de la quantifier de façon spécifique grâce à la mesure de l'accroissement de la fluorescence liée de la réaction de PCR.

La sonde permet de détecter l'ADN spécifique amplifié et de le quantifier par comparaison de l'intensité du signal avec celui du standard de quantification.

On entend ici par "amorce", un oligonucléotide de préférence de 15 à 25 nucléotides qui s'hybride de façon spécifique avec une des 2 extrémités de la séquence que l'ADN polymérase amplifiera dans la réaction de PCR.

Plus particulièrement, on met en oeuvre les jeux d'amorces et de sondes choisis le cas échéant parmi les séquences suivantes du listage de séquence annexé à la présente description, ou respectivement leurs séquences complémentaires :
- pour *Atopobium vaginae*:
   Amorce 5' : Seq. n°5 = 5'-CCCTATCCGCTCCTGATACC-3'
   Amorce 3' : Seq. n°6 = 5'-CCAAATATCTGCGCATTTCA-3'
   Sonde : Seq. n°7 = 5'-GCAGGCTTGAGTCTGGTAGGGGA-3'
      - pour *Gardnerella vaginalis*:
         Amorce 5' : Seq. n°8 = 5'-CGCATCTGCTAAGGATGTTG-3'
         Amorce 3' : Seq. n°9 = 5'-CAGCAATCTTTTCGCCAACT-3'
         Sonde : Seq. n°10 = 5'-TGCAACTATTTCTGCAGCAGATCC-3'
      - pour *Lactobacillus sp.:*
         Amorce 5' :Seq. n°11 = 5'-TACATCCCAACTCCAGAACG-3'
         Amorce 3' : Seq. n°12 = 5'-AAGCAACAGTACCCACGACCA-3'
         Sonde : Seq. n°13 = 5'-TGACAAGCCATTCTTAATGCA-3',
      - pour l'albumine humaine :
         Amorce 5' : Seq. n°14 = 5'-GCTGTCATCTCTTGTGGGCTGT-3'
         Amorce 3' : Seq. n°15 = 3'-AAACTCATGGGAGCTGCTGGTTC-3'
         Sonde :Seq. n°16 = 5'-CCTGTCATGCCCACACAAATCTCTCC-3'

De préférence, ledit grand fragment d'ADN synthétique constitutif de l'ADN de l'échantillon standard d'étalonnage de la quantification est inséré dans un plasmide.

Dans ces méthodes de quantification d'ADN, il est important de savoir si une réaction positive n'est pas due à une contamination par le plasmide recombinant utilisé comme standard de quantification ou comme témoin positif. Pour résoudre ce problème, un site de coupure par un enzyme de restriction est avantageusement introduit dans au moins une des cibles moléculaires synthétiques. Ce site étant absent sur la séquence naturelle. Donc, par coupure enzymatique et analyse du fragment amplifié sur gel d'agarose, ou en utilisant une sonde de PCR en temps réel qui reconnaît spécifiquement le site de restriction, on peut ainsi détecter la présence éventuelle du plasmide contaminant.

Ainsi, on peut mettre en oeuvre plus particulièrement, les étapes suivantes dans lesquelles :
1- on réalise une réaction d'amplification enzymatique de type PCR de l'ADN d'au moins une dite séquence spécifique d'au moins un desdits agents, dans l'ADN extrait desdits échantillons à tester et dans l'ADN de l'échantillon standard d'étalonnage, à l'aide d'au moins un jeu d'amorces apte à amplifier à la fois au moins ladite séquence spécifique authentique et ladite séquence spécifique modifiée,
2- on vérifie si les amplifiats éventuels de l'ADN extrait desdits échantillons à tester, comprennent une dite séquence spécifique, et
3- on détecte les faux positifs éventuels provenant de contaminations éventuelles desdits échantillons à tester par de l'ADN provenant de l'échantillon de standard d'étalonnage, par l'une au moins des étapes suivantes :
   - 3a- on réalise une digestion enzymatique du produit de PCR obtenu avec une enzyme correspondant au site de clivage et analyse sur gel d'agarose du produit de digestion en comparaison avec le produit de PCR non digéré par l'enzyme de restriction.

Si le fragment digéré provient de l'amplification de la cible moléculaire insérée dans le plasmide témoin, il contient le site de restriction, et il sera d'une taille inférieure au fragment non digéré.
- 3b- on réalise une réaction de type PCR en temps réel avec des amorces directes et inverses de l'une des cibles moléculaires, et une sonde spécifique de ladite séquence exogène contenant le site de restriction.

Seul un fragment provenant du plasmide témoin et contenant la séquence exogène pourra être amplifié.

Plus particulièrement, on met en oeuvre une dite séquence spécifique d'ADN humain dans l'échantillon à tester qui comprend le fragment des positions 16283-16423 de l'exon 12 du gène de l'albumine humaine de référence Genebank M12523.1 modifiée par insertion d'un site de clivage, notamment le site XhoI, en dehors des séquences correspondant aux amorces (séquences en gras) et séquence sonde (séquence soulignée) de séquence du listage de séquence suivante :
Seq. n°17 = 5'-**GCTGTCATCTCTTGTGGGCTGT**AATCATCGT(C TCGAG)TTAAGAGTAATATTGCAAAACCTGTCATGCCCACACAAATC TCTCCCTGGCATTGTTGTCTTTGCAGATGTCAGTGAAAGA**GAACCA GCAGCTCCCATGAGTTT**-3', comprenant le site XhoI (entre parenthèses) en dehors des séquences correspondant aux amorces (séquences en gras) et séquence sonde (séquence soulignée).

Avantageusement encore, on met en oeuvre une pluralité de réactions d'amplifications enzymatiques PCR simultanées ou non de chacune desdites séquences spécifiques desdites bactéries avec le même grand fragment d'ADN synthétique étalon, à l'aide d'une pluralité de différents jeux d'amorces spécifiques de chacune des différentes dites séquences spécifiques de chacune desdites bactéries, les séquences des différentes amorces ne se croisant pas entre les différentes dites bactéries et lesdites amorces pouvant être mises en oeuvre dans une réaction d'amplification enzymatique réalisée selon le même protocole et, notamment, à la même température d'hybridation.

On connaît différentes méthodes pour construire un grand fragment d'ADN chimère combinant plusieurs fragments, notamment d'origine diverses, notamment la méthode décrite dans FR 2 882 063 dans laquelle on prépare un grand premier fragment d'ADN synthétique double brin de séquence déterminée comprenant un enchaînement dans un ordre déterminé d'une pluralité de n deuxièmes petits fragments d'ADN synthétiques contigus, consistant essentiellement dans la dimérisation d'une pluralité de n oligonucléotides par amplification enzymatique à l'aide d'une enzyme polymérase thermorésistante comprenant :
- une première étape de réaction d'amplification d'acides nucléiques de type PCR en présence d'une dite enzyme polymérase, d'une série de n oligonucléotides de séquences déterminées, sans amorces exogènes, comprenant une série de cycles dans des conditions de températures permettant l'hybridation desdits oligonucléotides, suivie d'une élongation du complexe obtenu, destinée à mettre bout à bout dans un ordre déterminé lesdits oligonucléotides, les séquences desdits oligonucléotides correspondant successivement et alternativement aux séquences sens et anti-sens des différents dits deuxièmes fragments synthétiques, et chaque dit oligonucléotide contenant dans ses régions 5' et 3' des séquences complémentaires de celles des oligonucléotides suivant et précédent, le cas échéant, et
- une deuxième étape d'amplification à l'aide d'amorces spécifiques des extrémités 5' et 3' du dit brin directe dudit grand premier fragment synthétique à préparer, permettant de produire des copies identiques dudit grand premier fragment.

Cette technique est donc basée sur l'utilisation et la maîtrise d'un artefact de la PCR, qui consiste en l'hybridation des amorces entre elles (dimérisation des amorces). Ce phénomène est observé dans le cas où les conditions de PCR, notamment de température, sont mal adaptées, et que les amorces contiennent des séquences partiellement complémentaires.

La technique de construction consiste donc, à partir des séquences cibles, à choisir les séquences des oligonucléotides, avec une alternance d'oligonucléotides de séquences directes («sens») ou inverses (encore dénommées « reverses » ou «anti-sens»). Afin de rendre possible la mise bout à bout de ces oligonucléotides, on prend soin d'introduire en 3' de la séquence d'un oligonucléotide une séquence nucléotidique complémentaire des premiers nucléotides de l'oligonucléotide suivant. Ces oligonucléotides seront hybridés par leur parties complémentaires, et grâce à l'activité polymérasique, par exemple de la Taq polymérase, une synthèse de 5' en 3' se réalise afin d'obtenir des fragments doubles brins. Le fragment final (assemblé) est synthétisé par PCR en utilisant un couple d'amorces directe et inverse correspondant aux séquences des extrémités du premier grand fragment d'ADN synthétique bicaténaire désiré.

Comme mentionné précédemment, avantageusement, lesdits grands fragments d'ADN synthétiques, sont avantageusement insérés dans un plasmide.

Cette technique de construction générique d'un fragment nucléotidique synthétique permet la mise en contiguïté de plusieurs cibles moléculaires d'intérêt. Il s'agit d'une méthode simple à mettre en oeuvre, rapide et fiable, et ne nécessitant pas un équipement lourd et coûteux.

La présente invention a également pour objet une trousse de diagnostic utile pour la mise en oeuvre d'une méthode de diagnostic et suivi d'une vaginose selon l'invention, caractérisée en ce qu'elle comprend :
- des échantillons d'ADN standard d'étalonnage à une concentration connue comprenant desdites séquences spécifiques de chacun desdites bactéries tels que définis ci-dessus, et de préférence une dite séquence spécifique de l'ADN humain telle que définie ci-dessus, et de préférence encore un dit grand fragment d'ADN synthétique regroupant desdits séquences spécifiques de chacun desdites bactéries tels que définis ci-dessus, et de préférence une dite séquence spécifique de l'ADN humain telle que définie ci-dessus, ainsi que
- desdits jeux d'amorces spécifiques desdits fragments d'ADN synthétiques modifiés spécifiques desdites bactéries et de préférence encore, desdites sondes tels que définis ci-dessus, et
- des réactifs de mise en oeuvre d'une réaction d'amplification d'ADN de type PCR.

D'autres caractéristiques de la présente invention apparaîtront à la lumière de la description détaillée qui va suivre de différents exemples de réalisation en référence au listage de séquence et aux figures 1 à 4, dans lesquelles :
- la figure 1A représente l'analyse des charges microbiennes de 20 vaginoses bactériennes définies par le score de Nugent quantifiées par PCR en temps réel selon la présente invention.
- la figure 1B représente l'analyse des charges microbiennes de 167 flores normales définies par le score de Nugent quantifiées par PCR en temps réel selon la présente invention.
- la figure 2 représente l'analyse des charges microbiennes de 44 flores intermédiaires identifiées par le score de Nugent quantifiées par PCR en temps réel selon la présente invention.
- la figure 3 montre 25 vaginoses bactériennes identifiées sur des critères moléculaires et quantifiées selon la présente invention à partir du groupe des 44 flores intermédiaires définies par le score de Nugent.- la figure 4 présente 19 flores normales identifiées sur des critères moléculaires et quantifiées selon la présente invention après application des critères moléculaires de la vaginose bactérienne au groupe des 44 flores intermédiaires identifiées par le score de Nugent.

### I. PATIENTES, MATERIELS ET METHODES

### I.1 Réalisation et transport des prélèvements vaginaux

Les femmes enceintes, suivies pour leur grossesse, ont été recrutées à l'hôpital de La Conception à Marseille. Un consentement éclairé est la condition nécessaire à l'inclusion. Les prélèvements ont été réalisés au niveau du cul de sac vaginal postérieur sous spéculum stérile non lubrifié et sans antiseptique. Quatre prélèvements sont réalisés pour chaque femme : deux prélèvements par écouvillon coton sur tube sec (Copan innovation®, Brescia, Italie) et deux prélèvements par cytobrosse

(Scrinet® 5,5 mm, laboratoire C.C.D. international, Paris, France) Un écouvillon coton standard est utilisé pour l'état frais et la culture bactérienne. Un second est placé dans un milieu de transport spécifique (R1 Urée-Arginine LYO 2, BioMérieux SA, Marcy l'Etoile, France) pour la recherche des Mycoplasmes génitaux (*M. bominis* et *M. urealyticum*). Une cytobrosse est utilisée pour l'étalement sur lame et coloration de Gram. Une seconde pour l'extraction de l'ADN aux fins de quantification par amplification moléculaire est transportée dans 500 µl de milieu de transport MEM (Minimum Essential Médium, Invitrogen Life Technologies, Carlsbad, CA, USA). Elle est congelée à -80°C dés son arrivée au laboratoire jusqu'à son utilisation.

### I.2 Analyse bactériologique

### I.2.1 Etat frais

La recherche de *Trichomonas vaginalis* est réalisée par un examen à l'état frais entre lame et lamelle au microscope optique (objectif 10X).

### 1.2 Score de Nugent :

### I.2.2.1 La coloration de Gram :

Après étalement du prélèvement sur lame de verre et séchage la coloration de Gram comprend: une coloration au cristal violet oxalate (1 minute), une coloration au liquide de Lugol (1 minute), une décoloration à alcool/acétone et coloration au safranine en solution (BioMérieux). Chaque étape est suivie d'un lavage à l'eau. La coloration de Gram permet d'établir le score de Nugent selon une évaluation semi-quantitative de 3 morphotypes bactériens (Tableau 1). En fonction du score trois flores vaginales sont identifiées : une flore normale (FN) pour un score de 0 à 3, une flore intermédiaire (FI) pour un score de 4 à 6 et une VB pour un score supérieur à 7.

### I.2.2.2 Culture

Les prélèvements vaginaux sont ensemencés sur 3 milieux de culture : la gélose Columbia ANC plus 5% de sang de mouton (BioMérieux), la gélose Chocolat Poly Vitex PVX (BioMerieux), la gélose CHOC VCAT (BioMérieux) incubés à 37°C pendant 48 heures. Pour la recherche des Mycoplasmes, les prélèvements sont ensemencés sur kit spécifique (Urée-Arginine LYO 2, BioMérieux) puis incubés à 37°C puis inoculés sur milieux de culture anaérobie (BioMerieux) durant 48 heures.

### 1.3. Détection et quantification par PCR en temps réel :

### I.3.1 Extraction de l'ADN

L'extraction de l'ADN utilise le kit « QIAmp®DNA Mini Kit » (Qiagen®, Courtaboeuf, France). Le protocole est modifié de la façon suivante : incubation pendant 12 heures à 56°C de 200 µl d'échantillon pour 200 µl de tampon de lyse et 20 µl de protéinase K. Le lysat est traité selon les recommandations du fabriquant. L'ADN est élué dans 100 µl de tampon d'élution puis stocké à - 20°C.

### I.3.2 Mise au point des PCR en temps réel

### I.3.2.1 Choix des cibles moléculaires

L'analyse des données de la littérature, et des séquences déposées dans le site « GenBank » (http://www.ncbi.nlm.nih.gov/Genbank/GenbankSearch.html) permet de prendre connaissance des séquences disponibles pour chacun des microorganismes cibles. La spécificité des amorces, des sondes et des fragments des séquences cibles de chacun des microorganismes choisis sont testées pour leur spécificité sur le site Internet NBCI (http://WWW.ncbi.nlm.nih.gov/BLAST/).

Les inventeurs ont sélectionné des cibles sur tous les agents potentiels de vaginose, y compris des agents au rôle incertain. D'une façon surprenante, la cible la plus significative *Atopobium vaginae* n'était pas considérée comme un agent essentiel.

Les cibles retenues sont localisées sur la séquence du gène codant pour la protéine chaperonne de 60 KDa (*Cpn60*) pour *G*. *vaginalis* et *M. curtisii,* sur celle de l'ARN 16S pour *M. mulieris* et *A*. *vaginae,* le gène *fts Y* pour *M*. *bominis*, sur celle de l'uréase pour *U*. *urealyticum*, le gène de la topoisomérase III pour *C*. *albicans.* Pour la cible de *Lactobacillus sp.,* une séquence commune au: *Lactobacillus crispatus, Lactobacillus jensenii, Lactobacillus gasseri, Lactobacillus iners* et *Lactobacillus acidophilus* est choisie, elle est située sur le gène codant pour le facteur d'élongation *tu* (*tuf*). Une séquence située dans l'exon 12 du gène de l'albumine humaine est choisie afin d'attester de la présence et de la quantité d'ADN dans l'échantillon testé.

### 1.3.2.2 Choix des sondes et amorces

Pour chaque microorganisme étudié (*Lactobacillus sp., G. vaginalis, M. curtisii, M. mulieris, U. urealyticum, M. bominis, A. vaginae, C. albicans*) et l'albumine humaine une sonde, un couple amorce sens et anti-sens sont choisies sur les séquences cibles précédemment définies en utilisant le programme Primer 3® (http://frodo.wi.mit edu/primer3/primer3_code .html). Les amorces et sondes sont décrites dans l'annexe 1 ci- après Chaque amorce et sonde sont analysées sur le site Internet NBCI (http://WWW.ncbi.nlm.nih.gov/BLAST/) pour s'assurer de leur spécificité *in silico* (Annexe 1).

Afin de réaliser des PCR en temps réels en double reconnaissance (PCR duplex) des couples de reconnaissance de microorganismes sont arbitrairement choisies : une des sondes étant marquée FAM et l'autre étant marquée VIC. Quatre couples de PCR sont ainsi définis.
- *Lactobacillus sp.* (FAM) et *G*. *vaginalis* (VIC)
- *M. curtisii* (FAM) et *M*. *mulieris* (VIC)
- *U. urealyticum* (VIC) et albumine humaine (FAM)
- *A. v*aginae (VIC) et *C*. *albicans* (FAM)
- *Mycoplasma bominis* (FAM) sera quantifié seul.

Les amorces sont synthétisées par Eurogentec® (Seraing, Belgique), et les sondes par Applied Biosystems (Warrington, Cheshire, UK).

### I.3.2.3 Test de spécificité vis à vis des différentes souches testées

Afin de tester la spécificité des amorces et des sondes, l'ADN extrait de souches bactériennes de référence (*L*. *acidophilus* CIP 104464, *A*. *vaginae* CIP 106431, *G. vaginalis* CIP 103660, *M. curtisii* subsp, *bolmesii* ATCC 35242, *M. mulieris* ATCC 35239, *C. albicans* UMIP 1180.79, *U. urealyticum* CIP 103755 et *M. bominis* CIP 103715) est utilisé à trois dilutions (1/10^{e}, 1/100^{e}, 1/1000^{e}) pour la mise au point des PCR en temps réel (détermination des quantités relatives d'amorces et de sondes, des spécificités, des réactivités croisées). Chaque souche est testée avec les amorces et la sonde spécifique mais aussi avec les sondes et amorces des 7 autres microorganismes et celles de l'albumine humaine.

### I.3.2.4 Mise au point des PCR duplex

On a mesuré les valeurs de CT (« cycle threshold »). Il s'agit du point d'intersection entre la ligne de base de la réaction et la courbe logarithmique de représentation de l'amplification. Cette valeur de CT correspond au nombre de cycle d'amplification nécessaire pour que l'amplification commence. Elle est en relation avec la concentration du produit nucléique à quantifier, à savoir que plus le CT est bas plus la concentration est élevée.

Les quantités d'amorces et de sondes nécessaires pour l'obtention d'une amplification optimale en simple et en double fluorescence sont définies en testant les réactions d'amplification sur un mélange d'ADN bactérien des 8 souches de référence en équimolarité, ainsi qu'avec les souches pures, pour une concentration finale au 1/10^{e} dans les deux cas. Les conditions expérimentales retenues sont celles pour lesquelles les valeurs de CT sont identiques à celles obtenues en simple fluorescence.

### I.3.2.5 Test de spécificité vis à vis de 40 souches non testées :

Les échantillons d'ADN génomique provenant de 40 souches bactériennes différentes (Annexe) autres que les 8 souches bactériennes de référence sont testés en dilution au 1/10^{e}. L'ensemble de ces tests est effectué en prenant soin d'introduire lors de chaque réaction d'amplification des témoins négatifs ne contenant aucun ADN ou des témoins positifs (mélange de l'ADN des 8 souches de référence). La spécificité est testée avec les quatre couples de PCR en temps réel ainsi que *Mycoplasma bominis.*

### I.4. Construction du plasmide

On a mis en oeuvre la méthode de préparation décrite dans FR 2 882 063.

### 1.4.1 Séquence du fragment hybride de quantification :

La séquence nucléotidique du fragment hybride de quantification.est obtenue en alignant les séquences cibles des PCR en temps réel pour les 8 micro-organismes ainsi que celles de l'albumine humaine. Nous obtenons un fragment hybride de 931 paires de bases décrit à l'Annexe 2 ci- après.

### 1.4.2 Séquences des oligonucléotides de construction du fragment hybride de quantification :

La séquence du fragment nucléotidique hybride est divisée en 6 fragments d'oligonucleotides consécutifs décrits à l'Annexe 3. Afin d'assurer la mise en continuité des oligonucléotides adjacents, 10 nucléotides complémentaires de l'extrémité 3' sur l'oligonucléotide d'amont sont ajoutés à l'extrémité 5' de l'oligonucléotide d'aval. Les tailles des 6 séquences oligonucléotidiques s'échelonnent de 155 à 172 nucléotides. Les oligonucléotides consécutifs sont alternativement en séquence directe et reverse. Ainsi, les oligonucléotides 1, 3 et 5 sont utilisés sous forme de séquence directe, alors que les oligonucléotides 2, 4 et 6 sont utilisés en séquence reverse. Des amorces dites de construction (sens et antisens) dont les séquences correspondent à celles des 20 nucléotides en 5' et 3' du fragment hybride de quantification vont être synthétisées. Les oligonucléotides et les amorces sont synthétisés par Eurogentec®.

### I.4.3 Construction du fragment hybride :

Le fragment nucléotidique double brin est construit par réaction d'amplification grâce à la complémentarité des extrémités de deux oligonucléotides adjacents. Deux PCR successives sont nécessaires.

### I.4.3.1 Première PCR :

Elle permet l'hybridation des oligonucléotides par leurs extrémités et une élongation partielle quand elle est compatible avec l'activité de la Taq polymérase. Les 6 oligonucléotides sont introduits en équimolarité (0,2 mMol), avec du tampon de polymérase 1 X MgCl₂ (1,5 mMol), les dNTP (0,2 mMol), 0,2 µl de Taq Roche (Roche®) à 5 UI/µl, dans un volume réactionnel de 50 µl. Le programme d'amplification est : 95°C 2min, suivi de 40 cycles comportant 94°C 30 sec (dénaturation), 37°C 1 min (hybridation), 72°C 1 min 30 sec (élongation).

### I.4.3.2 Deuxième PCR :

Elle permet d'obtenir un fragment de PCR contenant bout à bout les motifs oligonuléotides attendus (Annexe 4). Un µl de produit d'amplification de la première PCR est ajouté au mélange réactionnel suivant : tampon de polymérase Hotstar (Qiagen®) 1 X MgCl₂ (1,5 mMol), les dNTP (0,2 mmol), 0,2 µl de Hotstar (Qiagen®) à 5 UI/µl, 0,2 mMol des amorces de construction sens et anti-sens. Le programme de PCR est : 95°C 15 min, suivi de 95°C 30s, 58°C 45s, 72°C 2min 40 cycles, 72°C 5min. Le fragment de PCR obtenu est analysé sur gel BET Agarose à 1,5% dans du tampon TBE 0,5X. Si la taille du fragment est conforme à la taille attendue, il est purifié en utilisant le kit QIAquick® PCR Purification Kit 250 PCR Qiakit (Qiagen®).

### I.4.3.3 Clonage de l'insert

Deux µl du fragment précédemment obtenu sont introduits dans un mélange réactionnel de ligation contenant 5µl de tampon de ligase, 1µl de ligase et 1µl de plasmide PGEM (kit pGEM®-T Easy Vector System 2 Promega ®, Madison, Wiconsin, USA) linéarisé et déphosphorylé. Le volume final est de 10µl. Le produit de ligation est incubé à 15°C pendant une nuit. Sept µl du produit de ligation sont mélangés à 50 µl de cellules compétentes (*Escherichia coli* JM 109) maintenues pendant 20 minutes dans la glace puis incubées pendant 1 minute à 42°C. Après ajout de 950 µl de LB broth (USB®, Cleveland, Ohio, USA), et incubation pendant 1h30 à 37°C, 500 et 250 µl de ce milieu de culture sont étalés sur 2 boites de Pétri contenant du LB agar (USB®) avec 100 µg/ml d'Ampicilline. Les boites sont incubées pendant une nuit à 37°C. Les colonies recombinantes sont déposées à la fois dans 50µl d'eau distillée stérile et sur une boite de LB agar Ampicilline.

Les colonies d'*E*. *coli* recombinantes sont prélevées pour être analysées par PCR : 5 µl de suspension bactérienne dans l'eau distillée, le couple d'amorces M13 (10 pm/µl) et le milieu réactionnel de PCR précédemment décrit. Le programme de PCR est identique à celui utilisé pour la deuxième étape de la construction. Les produits de PCR obtenus sont analysés sur gel d'Agarose à 1,5% dans du tampon TBE 0,5 X. Les fragments de la taille attendue sont purifiés en utilisant le kit Qiaquick® PCR Purification Kit 250 Qiakit (Qiagen®). Ils sont ensuite séquencés en utilisant le Big Dye® Terminator V1.1 Cycle Sequencing Kit (Applied Biosystems®, Warrington, UK), 3,2 pmol/µl d'amorces M13 sens et antisens chromatographiées sur le séquenceur ABI PRIM 3100 (Applied Biosystems®). La séquence obtenue est comparée à la séquence du fragment attendu en utilisant les programmes auto assembler et Sequence Navigator (Applied Biosystems®). Un des clones recombinants, conforme à la séquence attendue, va être produit en grande quantité dans 100 ml de LB BROTH Ampicilline et purifié selon le protocole High Speed Plasmid Midi Kit (Qiagen®). Le plasmide purifié est ensuite stocké à -20°C. La souche recombinante sélectionnée est congelée à - 80°C.

### I.4.3.4 Obtention de la gamme plasmidique

Sa concentration est déterminée par la mesure de la densité optique à 260 nm. Soit par exemple : 0,38 pour la solution initiale. 1 unité DO correspond à 50µl/ml : 0,38 x 50 = 19 µg/ml = 19. 10⁻⁶ g/ml de plasmide dans la solution initiale. La taille du plasmide = taille du pGEM®-T Easy + taille du fragment = 3015 + 931 = 4334 pb, soit 8668 bases. La masse molaire d'un nucléotide est de 330 Da (g/mol). La masse molaire du plasmide sera de 8668 x 330 = 2,860.10⁶ g/mol. La concentration du plasmide sera en mol/ml, soit 19.10⁻⁶ / 2,860.10⁶ = 6,64.10⁻¹² mol/ml. Multiplié par le nombre d'Avogadro, on obtient le nombre de copies de plasmide pour 1 ml de solution, soit : 6,64.10⁻¹² x 6,023. 10²³ = 40.10¹¹ copies/ml soit 2.10¹⁰ copies/5µl. Une première dilution de la solution plasmidique initiale au 1/2500^{e} permet d'ajuster la concentration au premier palier de la gamme plasmidique, soit le point 10⁷ copies/5µl de solution plasmidique. Une cascade de dilution par pas de 10 permet de créer les paliers successifs de la gamme (de 10⁷ copies à 1 copie pour 5µl de solution).

### 1.5. Analyse des échantillons

### I.5.1 PCR quantitative en temps réel

On réalise les réactions de quantification par PCR en temps réel sur les extraits d'ADN des prélèvements vaginaux. Au sein de chaque plaque de réaction, est introduit: 4 contrôles négatifs (NTC), la gamme plasmidique d'étalonnage (de 10⁷ à 1 copie par puits), 24 échantillons à tester, purs et dilués au 1/10^{e} et au 1/100^{e} pour rechercher des inhibiteurs. Les contrôles négatifs ainsi que les points de la gamme plasmique sont testés en double. Pour l'amplification et la quantification des 8 micro-organismes ainsi que de l'albumine humaine, on réalise 4 plaques de PCR en double fluorescence et une en simple fluorescence. Pour la préparation du mélange réactionnel, on utilise le kit « Quantitect Probe PCR Kit » (Qiagen®) contenant le mélange réactionnel 2X associant le tampon de la Taq polymérase et le Taq polymérase (Hotstar), les dNTP et dUTP. A ce milieu réactionnel, on ajoute les amorces sens et anti-sens et les sondes nécessaires à la réalisation de la PCR en simple ou en double fluorescence selon les conditions expérimentales rapportées décrites à l'Annexe 6, les prises d'essai des échantillons dilués ou non et des points de gamme plasmidique sont de5 µl et 0,25 µl d'UDG (Uracil DNA glycosylase à 100 Unités, Sigma-Aldrich, Lyon, France) est ajouté pour un volume réactionnel final de 25 µl. Les réactions de PCR sont réalisées sur l'appareil Stratagene® MX 3000P (La Jolla, California). Le programme d'amplification est le suivant: 2 minutes à 50°C, 15 minutes à 95°C, suivi de 45 cycles de PCR comportant la dénaturation à 95°C durant 30 secondes puis la phase d'hybridation et d'élongation à 60°C durant 1 minute.

### I.5.2 Calcul de la charge bactérienne

Pour chaque prélèvement vaginal et pour chaque microorganisme, afin d'assurer la comparabilité des échantillons, la charge bactérienne est définie de la façon suivante. La quantification en nombre de copies d'ADN de chaque micro-organisme pour 5 µl d'extrait d'ADN est rapportée en nombre de bactéries pour 1 ml d'échantillon initial. Il faut tenir compte du volume d'élution de l'ADN (100 µl), du volume du milieu de transport par échantillon initial (200 µl) et du fait que le gène quantifié est un gène unique. La valeur obtenue pour chaque microorganisme en nombre de copies pour 5 µl d'extrait d'ADN est multipliée par 10² pour obtenir une concentration en nombre de bactéries par ml d'échantillon, puisque l'on a réalisé un volume d'élution de 100 µl d'ADN extrait à partir de 200 µl d'échantillon.

### 1.5.3 Analyse statistique

L'analyse statistique des données de la quantification bactérienne par PCR en temps réel est réalisée en utilisant le test de Wilcoxon et le test de Mann-Whitney pour un le degré de signification p<0,05. Pour l'analyse statistique, toutes les valeurs de quantification sont prise en compte y compris celles au dessous du seuil de positivité. Pour les valeurs de quantification égale à zéro, la concentration la plus faible de l'ensemble des échantillons analysés leurs est attribuée. Le test statistique de Wilcoxon est appliqué pour décrire la répartition de chaque microorganisme au sein de chaque groupe de flore. Le test de Mann-Whitney est appliqué pour la comparaison de la quantification de chacun des 8 microorganismes dans le groupe des VB et des FN. Afin de rechercher des critères moléculaires de la VB, chaque seuil de quantification bactérienne (de 10³/ml à 10⁹/ml) est étudié d'après la sensibilité, la spécificité, la valeur prédictive positive et négative calculées selon l'identification par le seuil des FN et des VB préalablement définies par le score de Nugent. Les seuils de quantification, pris isolément ou de façon combinée, ayant la meilleure sensibilité et spécificité sont retenus comme critères moléculaires d'identification de la VB.

### II. RESULTATS

### II.1. Description de la population

De juin 2005 à avril 2006, 204 femmes enceintes âgées de 18 à 49 ans (moyenne d'age de 28,9 ± 6,2 ans) sont incluses. L'origine ethnique est l'Afrique du Nord (43%), l'Europe (42%), l'Afrique du Sud (14%) et d'autres origines (1 %). Chaque femme bénéficie d'un prélèvement vaginal. Soixante-douze pour cent des prélèvements sont réalisés au troisième trimestre contre 20% au second et 8% au premier trimestre de grossesse. Vingt-une femmes bénéficient d'un suivi bactériologie comportant 2 à 4 prélèvements. Un total de 231 prélèvements a ainsi été analysé.

### II.2. Résultats bactériologiques

### II.2.1 Coloration de Gram et établissement du score de Nugent

A partir des 231 prélèvements vaginaux provenant de 204 femmes, le score de Nugent permet d'identifier : 167 FN, 44 FI et 20 VB. La fréquence des anomalies de la flore vaginale en tenant compte du premier prélèvement vaginal pour chacune des 204 femmes est de 71% pour la FN (145 cas), 19% pour la FI (39 cas) et 10% pour la VB (20 cas). La moitié des femmes présentant une VB sont symptomatiques. Le symptôme le plus fréquemment observé est l'abondance des pertes vaginales.

### II.2.2 Culture et état frais

La culture permet d'isoler *G*. *vaginalis, C. albicans, M. bominis, U. urealyticum* ainsi que *Streptococcus agalatiae* (Tableau 1). Aucun des prélèvements vaginaux examinés ne présente de *T*. *vaginalis* à l'examen direct.

### II.3. Résultats de biologie moléculaire

### II.3.1 Mise au point de la PCR en temps réel

Lors de la mise au point des PCR en simple et en double fluorescence, on ne réalise aucune réaction croisée, aucune compétition. On obtient des résultats similaires en valeur de CT en simple et double fluorescence en utilisant les souches bactériennes pures et la gamme plasmidique.

Les quantités optimales d'amorces et de sondes nécessaires pour l'obtention d'une amplification optimale sont présentées en annexe 5.

### II.3.2 Evaluation de la technique de quantification par biologie moléculaire

La quantification bactérienne pour chaque échantillon est assurée par la gamme de dilution plasmidique. Pour chaque réaction d'amplification, les 8 points de la gamme plasmidique, de 10⁷ copies à 1 copie pour 5µl, sont testés. Le point de gamme 10⁷ copies est identifié de façon précoce pour un CT autour de 17. Pour le point de gamme, 1 copie la détection est plus tardive avec un CT à 37. Pour l'ensemble des points de la gamme, la réaction d'amplification est linéaire. La représentation graphique donne une droite dont la pente varie de -3,2 à -3,5. Cette linéarité est confirmée en testant les souches pures diluées au 1/10^{e}, 1/100^{e} et 1/1000^{e}. Un seuil de positivité est défini au dessus de 10 copies (soit 10³ bactéries/ml), quel que soit le type de microorganisme considéré (Tableau 2). En effet, de façon statistique, l'amplification du point « 1 copie » est assurée dans 75% alors qu'il est de 100% pour le point « 10 copies ». Au sein de chaque plaque d'amplification, chaque échantillon est testé en solution non diluée, diluée au dixième et au centième. De façon reproductible, la détection du produit d'amplification suit le pas de dilution, puisqu'une dilution au dixième correspond à une majoration de 3 CT. On considère uniquement la solution pure pour le calcul de la charge bactérienne Pour chaque échantillon, nous testons l'albumine humaine par PCR en temps réel. Les résultats obtenus sont homogènes pour l'ensemble des 231 échantillons. Les valeurs de CT se distribuent entre 19 et 22 (soit 10⁵ à 10⁶ copies d'ADN de l'albumine par 5 µl d'ADN extrait). Aucun échantillon n'a été exclu de l'analyse.

### II.3.3 Analyse des données

### II.3.3.1 Description moléculaire des vaginoses bactériennes

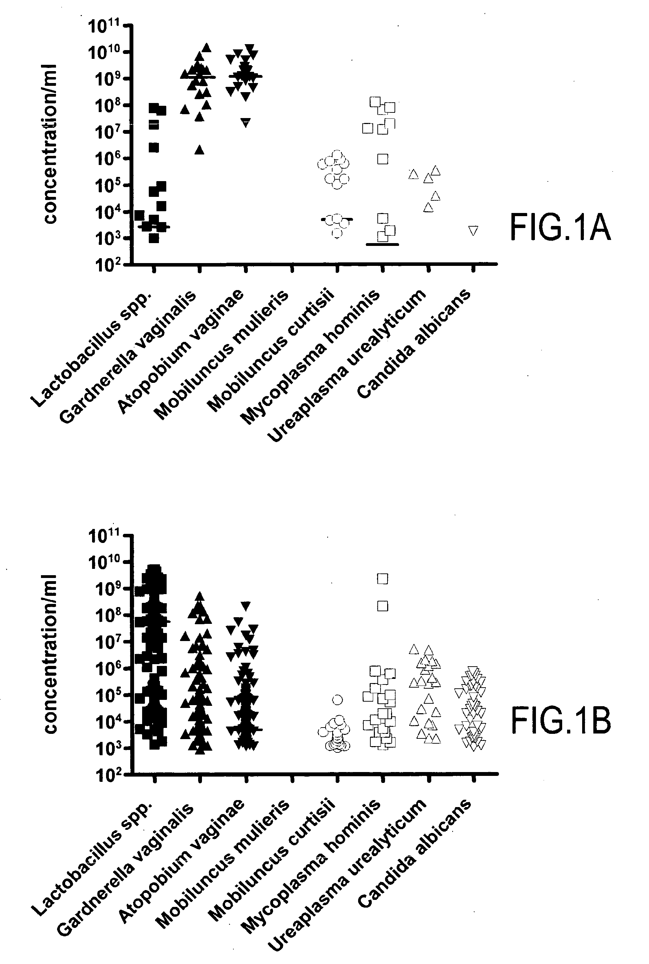

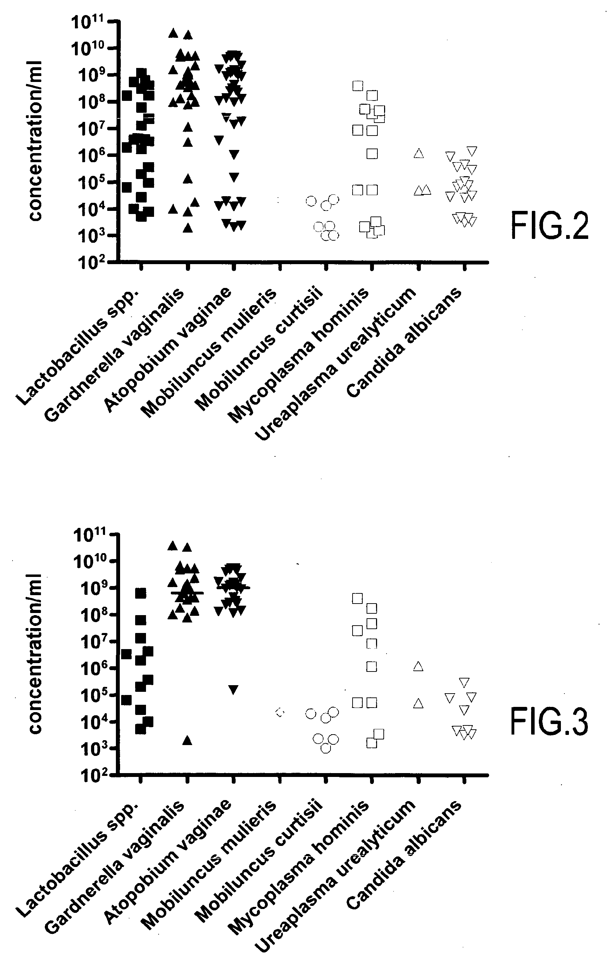

Les concentrations médianes d'*A. vaginae* et de *G*. *vaginalis* sont respectivement de 1,1.10⁹/ml et 1,2.10⁹/ml et n'ont pas de différence statistique significative par le test de Wilcoxon (*p*=0,3755). Ces 2 bactéries ont des concentrations élevées (Figures 1A et 3) par rapport aux autres microorganismes dont les concentrations médianes sont statistiquement plus basses (*p*=0,0001).

### II.3.3.2 Comparaison moléculaire des vaginoses bactériennes et des flores normales

La concentration médiane de *Lactobacillus sp*. est significativement plus basse (*p*<0,0001) pour les VB (concentration médiane de 3.10³/ml) que pour les FN (concentration médiane de 5,7.10⁷/ml). A l'opposé, les concentrations médianes d'*A*. *vaginae, G. vaginalis, M*. *curtisii* et *M*. *bominis* sont significativement plus élevées (*p*≤0,0037) pour les VB (concentration médiane respectivement de 1,1.10⁹ /ml; 1,2.10⁹ /ml; 5.10³ /ml et 5,5.10² /ml) que pour les FN. Pour les concentrations médianes d'*U*. *urealyticum* et *C. albicans,* il n'existe pas de différence statistique significative parmi les VB et les FN. Enfin, *M. mulieris* n'est identifié (seuil de positivité ≥10³/ml) dans aucune des VB ni aucune des FN.

### II.3.3.3 Définition des critères moléculaires de la vaginose bactérienne

L'analyse par seuil de la quantification pour *A*. *vaginae* et *G*. *vaginalis* pris isolément présente les meilleurs critères de sensibilité, spécificité, valeurs prédictives positive et négative pour l'identification moléculaire des VB et des FN définies par le score de Nugent (Tableau 4). L'association d'une quantification d'*A*. *vaginae* ≥10⁸/ml et/ou d'une quantification de *G. vaginalis* ≥10⁹/ml a une sensibilité de 95%, une spécificité de 99%, une valeur prédictive positive (VPP) de 95% et une valeur prédictive négative (VPN) de 99%.

### II.3.3.4 Caractérisation moléculaire des flores intermédiaires

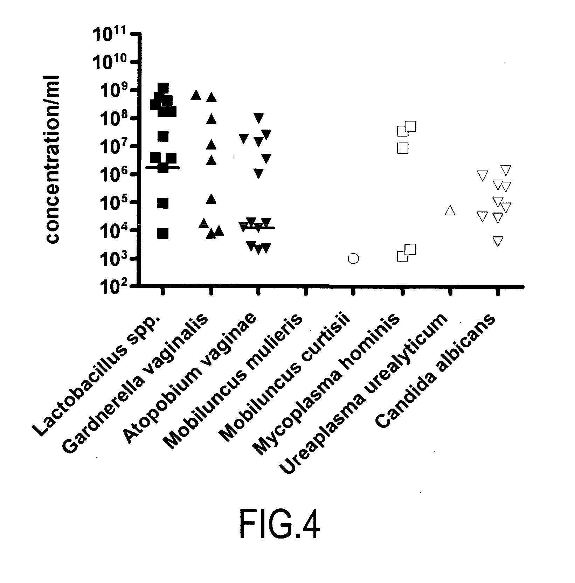

L'application aux FI du score de Nugent (Figure 2) des critères moléculaires d'identification de la VB précédemment définis par une quantification d'*A*. *vaginae* ≥10⁸/ml et/ou d'une quantification de *G*. *vaginalis* ≥10⁹/ml, permet de caractériser 25 flores (57%) présentant un profil de VB (Figure 3) et 19 flores (43%) présentant un profil de FN (Figure 4).

### II.3.3.5 Suivi bactériologique

Huit femmes présentant une VB ou une FI par le score de Nugent ont bénéficié d'un suivi (Tableau 7). La quantification moléculaire permet la mise en évidence de la récidive de la VB non identifiée par le score de Nugent pour le sujet 1. Elle permet de confirmer la disparition de la VB pour les sujets 7 et 8. Elle caractérise comme VB les FI du score de Nugent persistantes à plus d'un mois d'intervalle pour les sujets 2 et 5. Enfin, elle confirme le caractère normal du suivi de la flore vaginale du sujet 3.

### III DISCUSSION

La répartition des flores vaginales en FN (71%), VB (10%) et FI (19%) selon le score de Nugent est conforme à celles rapportées dans la littérature en France [Goffinet F, EJOGR 2003], en Europe [Guise JM, AMJPM 2001] et aux Etats-Unis [Delaney ML, OG 2001]. L'originalité de la présente invention est d'avoir établi un outil rationnel d'identification de la flore vaginale en associant la technique de détection spécifique par PCR en temps réel et la quantification relative par un plasmide d'étalonnage.

Le résultat le plus surprenant concerne *A*. *vaginae.* Cette bactérie a été identifiée pour la première fois en 1999 par amplification et séquençage de l'ARN 16S à partir d'un prélèvement vaginal de sujet sain [Rodriguez JM, IJSB 1999]. En 2003, *A*. *vaginae* a été isolée par culture dans un prélèvement d'abcès tubo-ovarien, laissant supposer un rôle pathogène de la bactérie [Geissdorfer W, JCM 2003]. En 2004, une approche d'amplification de l'ARN 16S r couplée à des techniques de clonage a permis la mise en évidence de la bactérie dans des prélèvements obtenus en per opératoire chez des patientes présentant une salpingite [Hebb JK, JID 2004]. Selon la présente invention, cette bactérie est d'identification fréquente dans 19 des 20 VB (95%) et 115 des 167 FN (69%). Le critère le plus discriminant pour le diagnostic des VB et des FN est une concentration d'*A*. *vaginae* ≥ 10⁸/ml avec une sensibilité de 90% (18 cas sur 20 VB) et une spécificité de 99% (1 cas sur 167 FN).

Depuis 2004, 4 études basées sur diverses techniques moléculaires ont mis en évidence une possible association entre *A*. *vaginae* et la VB mais aucune ne prenait en compte de critères de quantification rigoureux. Tout d'abord, une approche par PCR ciblant l'ARN 16S r suivi d'une migration sur gel a permis de mettre en évidence *A*. *vaginae* parmi seulement 12 VB sur 20 (60%) et 2 FN sur 24 (8,3%) [Ferris MJ, BMCID 2004]. Par PCR ciblant spécifiquement le gène de l'ARN 16S r d'*A*. *vaginae,* l'ADN de la bactérie a été retrouvée parmi 7 VB sur 9 (77,8%) et 22 FN sur 112 (19,6%) [Verhelst R, BMCM 2004]. En appliquant la même technique, l'ADN d'*A*. *vaginae* a été mis en évidence parmi 19 VB sur 22 (86,4%) et 59 FN sur 403 (14,7%) [Verhelst R, BMCM 2005]. Enfin par des techniques d'amplification, clonage et séquençage, l'ADN d'*A*. *vaginae* a été observé chez 26 VB sur 27 (96%) et 9 FN sur 46 (19,5%) [Fredricks DN, NEJM 2005]. Aucun de ces travaux n'a quantifié l'ADN d'*A*. *vaginae,* ce qui est essentiel dans les vaginoses bactériennes où la concentration des bactéries est un élément important du diagnostic.

*A*. *vaginae* fait défaut dans le score de Nugent malgré sa place capitale dans les anomalies de la flore vaginale. Son identification par des critères morphologiques est peu adaptée. Sa morphologie variable sous forme d'un petit coccobacille (0.6-0.9 µm) à Gram positif parfois regroupé par pair ou en courte chaîne lui assure un camouflage au contact d'autres bactéries, le rendant ainsi indétectable [Verhelst R, BMCM 2004]. Sa ressemblance aux *Lacfobacillus sp.* et aux streptocoques est donc source d'erreur d'identification [Rodriguez JM, 1JSB 1999].

La description d'une association entre *A. vaginae* et *G*. *vaginalis* dans la VB est récente, peu documentée et de portée diagnostique limitée en l'absence de quantification. Cette association est de 87,8% (8 sur 9 VB) [Verhelst R, BMCM 2004] et de 90% (20 sur 22 VB) [Zariffard MR, FEMS 2002] par PCR spécifique ciblant le gène de l'ARN 16S r d'*A*. *vaginae* et *G*. *vaginalis.* Une publication récente fait état de la présence d'*A*. *vaginae* dans les vaginoses bactériennes par une méthode semi-quantitative [Bradshaw CS, JID 2006].

Selon la présente invention, cette association d'*A*. *vaginae* et *G*. *vaginalis* est de 90% (18 sur 20 VB) mais la prise en compte de la quantification d'*A*. *vaginae* ≥10⁸/mL et/ou de *G*. *vaginalis* ≥10⁹/mL avec une sensibilité de 95% (19 sur 20 VB) présente les meilleurs critères de spécificité (99%), de VPP (95%) et de VPN (99%) jamais obtenu pour l'identification de la VB. En conséquence, on utilise une concentration d'*A*. *vaginae* ≥10⁸/mL et/ou une concentration de *G*. *vaginalis* ≥10⁹/mL pour le diagnostic moléculaire de la VB.

Les résultats selon la présente invention suggèrent que la quantification de *G*. *vaginalis* est moins discriminante que celle d'*A*. *vaginae.* Pour les *Lactolbacillus sp.,* leur diminution dans la VB objectivée par le score de Nugent est confirmée par ces résultats. En effet, si on considère une concentration de *Lactobacillus sp.* inférieure ou égale à 10⁷/ml, la sensibilité pour diagnostiquer une VB est de 100% et la spécificité de 44%. D'autre part, si nous considérons une concentration de *Lactobacillus sp.* supérieure ou égale à 10⁸/ml, nous observons une spécificité de 100% pour mettre en évidence une flore vaginale normale.

Pour les *Mobiluncus* spp., malgré la place qui leur est accordée par le score de Nugent, aucune PCR n'est positive pour *M*. *mulieris.* Seule *M*. *curtisii* est associée à la VB. Cependant, son utilisation comme possible critère diagnostique moléculaire reste mineure.

Les mycoplasmes génitaux ne sont pas pris en compte par le score de Nugent. Ils restent identifiables par culture ou biologie moléculaire. L'étude par culture de 445 VB et 2729 FN, identifiait *M. bominis* comme significativement associé à la VB avec une prévalence de 29% (129 VB) et *U. urealyticum* comme non associé à la VB malgré une prévalence de 56% (253 VB) [Thorsen P, AJOG 1998]. Une étude sur 203 VB et 203 FN ciblant *M*. *bominis* par PCR en temps réel, suggérait une implication de *M*. *bominis* dans la VB [Zariffard MR, FEMS 2002]. Cependant, une étude identique sur un effectif plus restreint (5 VB et 16 FN) ne mettait pas en évidence ce lien [Sha BE, JCM 2005]. Selon la présente invention, seul *M*. *bominis* est corrélé à la VB mais cette corrélation est insuffisante pour proposer ce microorganisme comme critère diagnostique de la VB.

Les résultats selon la présente invention ne mettent pas en évidence de lien entre la VB et *C*. *albicans* comme le montre les données de la littérature [Thorsen P, AJOG 1998]. Ceci est intéressant car si le portage génital à *Candida albicans* ne majore pas le risque de prématurité [Cotch MF, AJOG 1998], une étude récente [Kiss H, BMJ 2004] a montré une réduction du taux de prématurité par le traitement du portage génital à *C*. *albicans*.

L'originalité de la présente invention est de pouvoir proposer pour la première fois un outil diagnostic de la VB basé sur la quantification d'*A*. *vaginae* et *G*. *vaginalis.* Les critères retenus actuellement associent une concentration d'*A*. *vaginae* ≥10⁸/mL et/ou une concentration de *G*. *vaginalis* ≥10⁹/mL. Ce test diagnostique a une sensibilité de 95% une spécificité de 99%, une VPP de 95% et une VPN de 99%. Plusieurs méthodes de lecture des résultats de PCR, notamment celles par calcul de ratio et de produits de concentrations bactériennes, ont été testées pour permettre d'identifier la méthode la plus pertinente.

L'une des applications la plus troublante de l'outil de biologie moléculaire selon la présente invention est celle réalisée sur la FI du score de Nugent. Nous savons que la FI, uniquement identifiée par le score de Nugent, constitue une fraction non négligeable (8% à 22%) de la totalité des flores identifiées par ce dernier [Guerra B, EJOGRB 2006 ; Goffinet F, EJOGRB 2003 ; Delaney ML, OG 2001 ; Libman MD, DMID 2006 ; Larsson PG, STI 2004] et qu'elle est associée à de nombreuses incertitudes. En effet, son interprétation est sujet à caution car nous ne savons pas à quelle réalité microbiologique cette flore intermédiaire correspond. Certains la considèrent comme une flore de transition entre FN et VB [Ison CA, STI 2002 ; Guerra B, EJOGRB 2006 ; Goffinet F, EJOGRB 2003 ;Ugwumadu A, Lancet 2003 ; Carey JC, NEJM 2000]. D'autres la considèrent comme un groupe hétérogène regroupant des FN et des VB [Ison CA, STI 2002 ; Libman MD, DMID 2006 ; Larsson PG, STI 2004]. Des tentatives de caractérisation de la FI en FN ou VB sont rapportées dans la littérature. Par l'application des critères d'Amsel à 13 FI, 12 FI sont reclassées en FN et 1 en VB [Ison CA, STI 2002]. Par la coloration de Kopeloff pour l'établissement du score de Nugent, 69 des 232 FI (30%) sont reclassés en FN ou VB [Libman MD, DMID 2006]. Dans cette coloration, la fuchsine remplace la safranine de la coloration de Gram pour permettre une meilleure identification des bactéries à Gram négatif. Enfin, la standardisation des critères du score de Nugent en fonction de la surface du champ optique de l'appareil microscopique utilise, a permis de reclasser 458 des 1176 FI (39%) en FN ou VB [Larsson PG, ST1 2004].

L'application aux FI des critères moléculaires selon la présente invention associant à la VB une concentration d'*A. vaginae* ≥10⁸/ml et/ou d'une concentration de *G. vaginalis* ≥10⁹/ml permettent d'envisager une caractérisation rationnelle de cette FI. Ainsi, 24 FI (57%) présentent un profil similaire à celui des VB. Les dix-neuf autres FI (43%) présentent un profil similaire à celui des FN. Ces résultats laissent donc suggérer d'une part le caractère hétérogène des FI et d'autre part un défaut de sensibilité du score de Nugent pour diagnostiquer plus de la moitié des VB. Ces données confirment donc les limites du score de Nugent.

L'étiologie de la VB reste encore bien mystérieuse mais on dispose pour la première fois d'un outil de quantification objectif permettant une approche diagnostique rationnelle de la VB. Le caractère singulier de la présente invention est d'avoir montré à la fois la position capitale d'*A, vaginae* dans la VB mais aussi son utilisation par quantification comme critère diagnostique principal de la VB. On comprend d'autant mieux le problème thérapeutique soulevé par le caractère relativement résistant d'*A. vaginae* au métronidazole pouvant expliquer en partie la fréquence des récidives après traitement [ANAES 2001 ; Ferris MJ , BMC ID 2004 ; Secor AM, CNP 1997 ; Geissdorfer W, JCM 2003 ; De Backer E, BMC ID 2006]. Les critères moléculaires retenus pour le diagnostic de la VB associent une concentration d'*A. vaginae ≥*10⁸/ml et/ou d'une concentration de G. *vaginalis* ≥10⁹/ml. Cet outil moléculaire permet le diagnostic de la VB et la caractérisation en VB d'une fraction de la FI. Nous pouvons aussi envisager notre outil moléculaire comme une méthode diagnostique de la VB et de suivi pour l'évaluation la prise en charge thérapeutique de la VB durant la grossesse.

### Annexe 1. Cible moléculaire et séquence nucléotidique du couple d'amorces et de la sonde pour chaque microorganisme étudié.

Dans le tableau ci-dessous, les séquences des «amorces sens » et « sondes » sont écrites dans le sens 5'->3', et les séquences des « amorces anti-sens» sont écrites dans le sens 5'->3' complémentaire inversé

| **Micro-organismes** | **Cibles** | **Séquences nucléotidiques** |
|---|---|---|
| *M. curtisii* | *Cpn 60* | Amorce sens: TGGAAAAGGTGGGTCAAGAG Amorce anti-sens: AAACGCATACCTTCGGTGAC Sonde : FAM-GGCGTCATCACCGTGGAAGAA-TAMRA |
| *M. mulieris* | ARN 16 S ribosomal | Amorce sens : ATGGATATGCGTGTGGATGG Amorce anti-sens : CCAGGCATGTAAGCCCAAAC Sonde : VIC-TTTTGGGTGGGGGCGCTA-TAMRA |
| *G. vaginalis* | *Cpn 60* | Amorce sens : CGCATCTGCTAAGGATGTTG Amorce anti-sens : CAGCAATCTTTTCGCCAACT Sonde: VIC-TGCAACTATTTCTGCAGCAGATCC-TAMRA |
| *Lactobacillus sp.* | *Tuf* | Amorce sens: TACATCCCAACTCCAGAACG Amorce anti-sens : AAGCAACAGTACCACGACCA Sonde : FAM-TGACAAGCCATTCTTAATGCCA-TAMRA |
| *U. urealyticum* | Uréase | Amorce sens: ACTGGTGACCGTCCTATCCA Amorce anti-sens : CCTGATGGAATATCGAAACGA Sonde:VIC-TGAAAAAGGAAACGAAGACAAAGA-TAMRA |
| *M. hominis* | *fts Y* | Amorce sens : ATTGATTGCTGCAGGTGATACA Amorce anti-sens : GGTGTTACAATATCAGCCCCAAC Sonde : FAM-AGAGCAGCGGCAGTTGAA-TAMRA |
| *A. vaginae* | ARN 16S ribosomal | Amorce sens : CCCTATCCGCTCCTGATACC Amorce anti-sens : CCAAATATCTGCGCATTTCA Sonde:VIC-GCAGGCTTGAGTCTGGTAGGGGA-TAMRA |
| *C. albicans* | Topoisoméras e 3 | Amorce sens : CAACGCCAACGAAGACAAG Amorce anti-sens : CCAGCTTTGTTTGCATCAAC Sonde : FAM-AAAGCCGATGGTAGTAGAAAACTGCTAMRA |
| Albumine humaine | Exon 12 | Amorce sens : GCTGTCATCTCTTGTGGGCTGT Amorce anti-sens : AAACTCATGGGAGCTGCTGGTTC Sonde : FAM-CCTGTCATGCCCACACAAATCTCTCC-TAMRA |

### Annexe 2. Séquence théorique globale de l'insert de 939 paires de bases.

Séquences en gras = Séquences des amorces sens et anti-sens
Séquences en souligné = Séquences des sondes spécifiques
Séquences en noir = Séquences intercalentes
Séquences entre parenthèses= Séquences du site de restriction Xhol

### Annexe 3. Séquences des 6 oligonucléotides et des amorces de construction de l'insert.

- Oligonucleotide 1 sens, 178 nucléotides: séquences de *M. curtisii* et *M. mulieris*
- Oligonucléotide 2 anti-sens, 183 nucléotides: *G. vaginalis* et *Lactobacillus sp.* - Oligonucléotide 3 sens, 145 nucléotides: *U. urea*/*yticum* - Oligonucléotide 4 anti-sens, 188 nucléotides: *M. bominis* - Oligonucléotide 5 sens; 152 nucléotides: *A. vaginae* et *C. albicans* - Oligonucléotide 6 anti-sens, 147 nucléotides: albumine humaine
- Amorces de construction pour le plasmide recombinant avec le fragment de 939 paires de bases
- Sens Seq. n°25=5'GCCATGGAAAAGGTGGGTC3'
   Anti-sens Seq. n°28=5'CCAAACTCATGGGAGCTGCT3'

### Annexe 4. Schémas théoriques de construction d'un insert par double PCR.

Le principe de construction d'un insert avec 6 oligonucléotides.

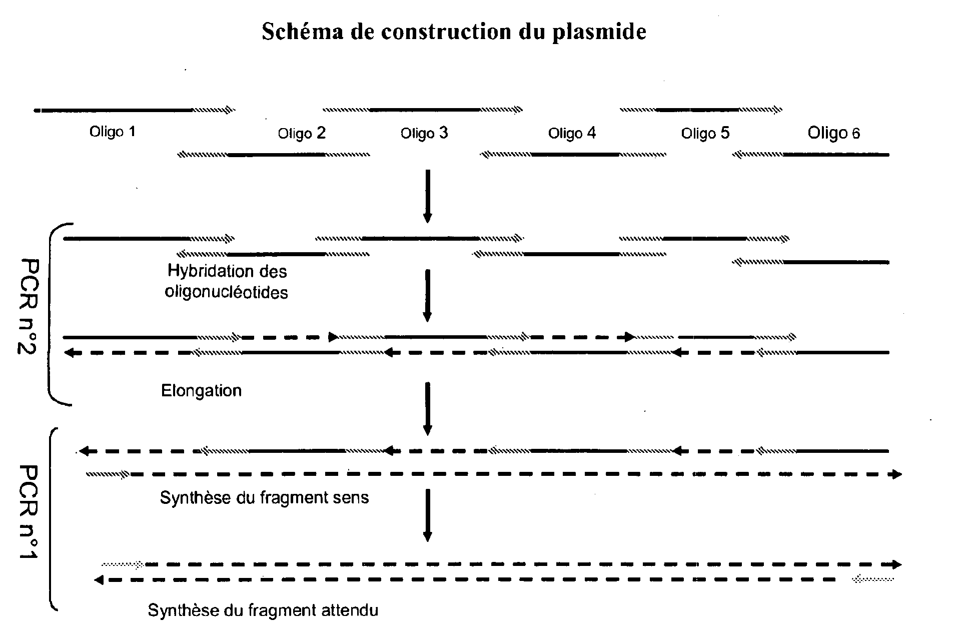

### Annexe 5. Quantité de sonde et d'amorces sens et anti-sens nécessaire à la réaction d'amplification de 5µl d'extrait d'ADN pour chaque micro-organisme et l'albumine humaine.

| Micro-organismes | Sonde (quantité exprimée en µl par puits) | Amorce sens (quantité exprimée en µl par puits) | Amorce anti-sens (quantité exprimée en µl par puits) |
|---|---|---|---|
| *M. curtisii* | 0,25 | 0,5 | 0,5 |
| *M. mulieris* | 0,25 | 0,5 | 0,5 |
| *G. vaginalis* | 0,25 | 0,5 | 0,5 |
| *Lactobacillus sp*. | 0,25 | 0,5 | 0,5 |
| *U. urealyticum* | 0,25 | 0,5 | 0,5 |
| *M. bominus* | 0,125 | 0,125 | 0,125 |
| *A. vaginae* | 0,25 | 0,5 | 0,5 |
| *C. albicans* | 0,25 | 0,5 | 0,5 |
| Albumine humaine | 0,125 | 0,125 | 0,125 |

### BIBLIOGRAPHIE

ANAES. 2001. Prévention anténatale du risque infectieux bactérien anténatal. Recommandations pour les professionnels de santé.
Amsel R, Totten PA, Spiegel CA, Chen KC, Eschenbach D, Holmes KK. Nonspecific vaginitis. Diagnostic criteria and microbial and epidemiologic associations. Am J Med. 1983;74:14-22.
De Backer E, Verhelst R, Verstraelen Hans, Antibiotic susceptibility of Atopobium vaginae. BMC infectious diseases, March 2006.
Bradshaw CS, Morton AN, Hocking J. High recurrence rates of bacterial vaginosis over the course of 12 months after oral metronidazole therapy and factors associated with récurrence. J Infect Dis 2006; 193:1478-86.
Bradshaw CS, Tabrizi SN, Fairley CK, Morton AN, Rudland E, Garland SW. The association of Atopobium vaginae and Gardnerella vaginalis with bacterial vaginosis and recurrence after oral metronidazole therapy. J Infect Dis. 2006; 194:828-36.
Carey JC, Klebanoff MA, Hauth JC, Hillier SL, Thom EA, Ernest JM, Heine RP, Nugent RP, Fischer ML, Leveno KJ, Wapner R, Varner M. Metronidazole to prevent preterm delivery in pregnant women with asymptomatic bacterial vaginosis. National Institute of Child Health and Human Development Network of Maternal-Fetal Medicine Units. N Engl J Med. 2000 ;342:534-40.
Cotch MF, Hillier SL, Gibbs RS, Eschenbach DA. Epidemiology and outcomes associated with moderate to heavy Candida colonization during pregnancy. Vaginal Infections and Prematurity Study Group. Am J Obstet Gynecol. 1998;178:374-80.
Delaney ML, Onderdonk AB. Nugent score related to vaginal culture in pregnant women. Obstet Gynecol. 2001;98:79-84.
Ferris MJ, Masztal A, Aldridge KE, Fortenberry JD, Fidel PL Jr, Martin DH. Association of Atopobium vaginae, a recently described metronidazole résistant anaerobe, with bacterial vaginosis. BMC Infect Dis. 2004 Feb 13;4:5.
Ferris, M. J., A. Masztal, and D. H. Martin. Use of species-directed 16S rRNA gene PCR primers for détection of Atopobium vaginae in patients with bacterial vaginosis. J Clin Microbiol. 2004; 42:5892-4.
Fredricks DN, Fiedler TL, Marrazzo JM. Molecular identification of bacteria associated with bacterial vaginosis. N Engl J Med. 2005; 353:1899-911.
Geissdorfer W, Bohmer C, Pelz K, Schoerner C, Frobenius W, Bogdan C Tuboovarian abscess caused by Atopobium vaginae following transvaginal oocyte recover. J Clin Microbiol. 2003;41:2788-90.
Goffinet F, Maillard F, Mihoubi N, Kayem G, Papiernik E, Cabrol D, Paul G. Bacterial vaginosis: prevalence and predictive value for premature delivery and neonatal infection in women with preterm labour and intact membranes. Eur J Obstet Gynecol Reprod Biol. 2003;108:146-51.
Guerra B, Ghi T, Quarta S, Morselli-Labate AM, Lazzarotto T, Pilu G, Rizzo N. Pregnancy outcome after early detection of bacterial vaginosis. Eur J Obstet Gynecol Reprod Biol. 2006 Feb 2.
Guise JM, Mahon SM, Aickin M, Helfand M, Peipert JF, Westhoff C. Screening for bacterial vaginosis in pregnancy. Am J Prev Med. 2001 ;20(3 Suppl):62-72.
Hauth JC, Goldenberg RL, Andrews WW, DuBard MB, Copper RL. Reduced incidence of preterm delivery with metronidazole and erythromycin in women with bacterial vaginosis. N Engl J Med. 1995;333:1732-6.
Hebb JK, Cohen CR, Astete SG, Bukusi EA, Totten PA. Détection of novel organisms associated with salpingitis, by use of 16S rDNA polymerase chain reaction. J Infect Dis. 2004;190:2109-20.
Ison CA, Hay PE. Validation of a simplified grading of Gram stained vaginal smears for use in genitourinary medicine clinics. Sex Transm Infect. 2002;78:413-5.
Kiss H, Petricevic L, Husslein P. Prospective randomised controlled trial of an infection screening programme to reduce the rate of preterm delivery. BMJ. 2004;329:371.
Larsson PG, Carlsson B, Fahraeus L, Jakobsson T, Forsum U. Diagnosis of bacterial vaginosis: need for validation of microscopic image area used for scoring bacterial morphotypes. Sex Transm Infect. 2004;80:63-7.
Leitich H, Brunbauer M, Bodner-Adler B, Kaider A, Egarter C, Husslein P. Antibiotic treatment of bacterial vaginosis in pregnancy: a meta-analysis. Am J Obstet Gynecol. 2003;188:752-8.
Libman MD, Kramer M, Platt R; Montreal Prematurity Study Group. Comparison of Gram and Kopeloff stains in the diagnosis of bacterial vaginosis in pregnancy. Diagn Microbiol Infect Dis. 2006;54:197-201.
McDonald H, Brocklehurst P, Parsons J. Antibiotics for treating bacterial vaginosis in pregnancy. Cochrane Database Syst Rev. 2005 Jan 25;(1):CD000262. Review.
Morales HJ, Schorr S and J Albritton, Effect of metronidazole in patients with preterm birth in preceding pregnancy and bacterial vaginosis: a placebo-controlled, double-blind study, Am J Obstet Gynecol 171 (1994), pp. 345-347.
Nugent RP, Krohn MA, Hillier SL. Reliability of diagnosing bacterial vaginosis is improved by a standardized method of gram stain interpretation. J Clin Microbiol. 1991;29:297-301.
Okun N, Gronau KA, Hannah ME. Antibiotics for bacterial vaginosis or Trichomonas vaginalis in pregnancy: a systematic review. Obstet Gynecol. 2005;105:857-68.
Rodriguez Jovita M, Collins MD, Sjoden B, Falsen E. Characterization of a novel Atopobium isolate from the human vagina: description of Atopobium vaginae sp. nov. Int J Syst Bacteriol. 1999;49:1573-6.
Schwebke JR, Hillier SL, Sobel JD, McGregor JA, Sweet RL. Validity of the vaginal gram stain for the diagnosis of bacterial vaginosis. Obstet Gynecol. 1996;88:573-6.
Schwebke JR, Lawing LF. Prevalence of Mobiluncus spp among women with and without bacterial vaginosis as detected by polymerase chain reaction.Sex Transm Dis. 2001;28:195-9.
Secor, A. M. 1997. Diagnosis and treatment of chronic vulvovaginitis. Clin Nurse Practionner 1:1-6.
Sha BE, Chen HY, Wang QJ, Zariffard MR, Cohen MH, Spear GT. Utility of Amsel criteria, Nugent score, and quantitative PCR for Gardnerella vaginalis, Mycoplasma bominis, and Lactobacillus sp. for diagnosis of bacterial vaginosis in human immunodeficiency virus-infected women. J Clin Microbiol. 2005;43:4607-12
Spiegel CA., Bacterial Vaginosis. Clinical Microbiology Reviews. 1991; 4:485-502.
Thomason JL, Anderson RJ, Gelbart SM, Osypowski PJ, Scaglione NJ, el Tabbakh G, James JA. Simplified gram stain interpretive method for diagnosis of bacterial vaginosis. Am J Obstet Gynecol. 1992;167:16-9.
Thorsen P, Jensen IP, Jeune B, Ebbesen N, Arpi M, Bremmelgaard A, Moller BR. Few microorganisms associated with bacterial vaginosis may constitue the pathologie core: a population-based microbiologie study among 3596 pregnant women. Am J Obstet Gynecol. 1998;178:580-7.
Ugwumadu A, Manyonda I, Reid F, Hay P. Effect of early oral clindamycin on late miscarriage and preterm delivery in asymptomatic women with abnormal vaginal flora and bacterial vaginosis: a randomised controlled trial. Lancet. 2003;361:983-8.
Varma R, Gupta JK. Antibiotic treatment of bacterial vaginosis in pregnancy: multiple meta-analyses and dilemmas in interprétation. Eur J Obstet Gynecol Reprod Biol. 2006;124:10-4.
Verhelst, R., H. Verstraelen, G. Claeys, G. Verschraegen, J. Delanghe, L. Van Simaey, C. De Ganck, M. Temmerman, and M. Vaneechoutte. 2004. Cloning of 16S rRNA genes amplified from normal and disturbed vaginal microflora suggests a strong association between Atopobium vaginae, Gardnerella vagina/is and bacterial vaginosis. BMC.Microbiol 2004; 4:16.
Verhelst R, Verstraelen H, Claeys G, Verschraegen G, Van Simaey L, De Ganck C, De Backer E, Temmerman M, Vaneechoutte M. Comparison between Gram stain and culture for the characterization of vaginal microflora: definition of a distinct grade that resembles grade I microflora and revised categorization of grade I microflora. BMC Microbiol. 2005 Oct 14; 5:61.
Zariffard MR, Saifuddin M, Sha BE, Spear GT. Détection of bacterial vaginosis-related organisms by real-time PCR for Lactobacilli, Gardnerella vaginalis and Mycop/asma bominis. FEMS Immunol Med Microbiol. 2002 13;34:277-81.

**Tableau. 1 Résultats de la culture bactérienne pour les 3 types de flore identifiés par le score de Nugent.**

| Culture | Flore normale | Flore intermédiaire | Vaginose bactérienne | Total |
|---|---|---|---|---|
| Micro-organisme recherché | Nombre de prélèvements positifs sur nombre de prélèvements Testés (%) | | | |
| *G. vaginalis* | 6 sur 167 (3,6%) | 6 sur 44 (13,6%) | 15 sur 20 (75%) | 27 sur 231 (12%) |
| *M. bominis* et *U. urealyticum* | 16 sur 163 (9,8%) | 6 sur 39 (15%) | 4 sur 16 (25%) | 26 sur 2 (9%) |
| *C. albicans* | 30 sur 167 (18%) | 17 sur 44 (38,6%) | 3 sur 20 (15%) | 50 sur 231 (21,6%) |
| *Streptococcus agalactiae* | 5 sur 167 (3%) | 1 sur 44 (2%) | 0 sur 20 | 6 sur 231 (2,6%) |

**Tableau 2. Détection de la présence des 8 microorganismes par PCR en temps réel pour les 231 échantillons.**

| Micro-organismes ciblés par PCR | Nombre et pourcentage de PCR positive (seuil de positivité 10³/ml) |
|---|---|
| *Lactobacillus sp.* | 177 (77%) |
| *G. vaginalis* | 131 (57%) |
| *A. vaginae* | 172 (74%) |
| *M*. *mulieris* | 1 (0,4%) |
| *M. curtisii* | 41 (18%) |
| *M. bominis* | 47 (20%) |
| *U. urealyticum* | 32 (14%) |
| *C. albicans* | 60 (26%) |

**Tableau 3. Analyse des concentrations bactériennes médianes obtenues par PCR quantitative pour les vaginoses bactériennes et les flores vaginales normales déterminées par le score de Nugent.**

| Micro-organismes | 20 vaginoses bactériennes | 167 flores normales | *P* value |
|---|---|---|---|
| | Concentration bactérienne médiane/ml (concentration minimale et maximale) | | Test statistique de Mann-Whitney |
| *Lactobacillus sp.* | 2,7.10³ (<10³-8.10⁷) | 5,6.10⁷ (<10³-5,4.10⁹) | < 0.0001 |
| *G. vaginalis* | 1,1 10⁹ (<10³-1,5.10¹⁰) | <10³(<10³-5.10⁸) | < 0.0001 |
| *A. vaginae* | 1,2 10⁹ (<10³-1,2.10¹⁰) | 4,7.10³ (<10³-2.10³) | < 0.0001 |
| *M. mulieris* | <10³ | <10³ | non mesurable |
| *M*. *curtisii* | 4,9.10³ (<10³-1,3.10⁶) | <10³ (<10³-6,2.10⁴) | < 0.0001 |
| *M*. *bominis* | 8,5.10² (<10³-13.10⁸) | <10³ (<10³-2,2.10⁹) | 0.0054 |
| *U. urealyticum* | <10³ (<10³-3,4.10⁵) | <10³ (<10³-5.10⁶) | 0,3344 |
| *C. albicans* | <10³ (<10³-1,7.10³) | <10³(<10³-7.10⁶) | 0,0991 |

**Tableau 4. Impact de la quantification bactérienne par PCR en temps réel pour l'identification des vaginoses bactériennes.**

| Le groupe de flores normales et des vaginoses bactériennes définies par le score de Nugent sont utilisés comme référence. | | | | | | | |
|---|---|---|---|---|---|---|---|
| Micro-organismes | Seuil de quantification (bactéries/ml) | FN identifiées* | VB identifiées* | Sensibilité** | Spéciticité** | VPP*** | VPN*** |
| *A. vaginae* | ≥ 10³ | 116 (69%) | 19 (95%) | 0,95 | 0,31 | 0,14 | 0,98 |
| | ≥ 10⁴ | 65 (39%) | 19 (95%.) | 0,95 | 0,61 | 0,23 | 0,99 |
| | ≥ 10⁵ | 25 (15%) | 19 (95%.) | 0,95 | 0,85 | 0,43 | 0,99 |
| | ≥ 10⁶ | 13 (7,8%) | 19 (95%.) | 0,95 | 0,92 | 0,59 | 0,99 |
| | ≥ 10⁷ | 6 (3,6%) | 19 (95%) | 0,95 | 0,96 | 0,76 | 0,99 |
| | ≥ 10⁸ | 1 (0,6%) | 18 (90%) | 0,90 | 0,99 | 0,95 | 0,99 |
| | ≥ 10⁹ | 0 | 13 (65%) | 0,65 | 1,00 | 1,00 | 0,96 |
| *G.* vaginalis | ≥ 10³ | 79 (47%) | 19 (95%) | 0,95 | 0,53 | 0,19 | 0,99 |
| | ≥ 10⁴ | 59 (35%) | 19 (95%) | 0,95 | 0,65 | 0,24 | 0,99 |
| | ≥ 10⁵ | 39 (23%) | 19 (95%) | 0,95 | 0,77 | 0,33 | 0,99 |
| | ≥ 10⁶ | 25 (15%) | 19 (95%) | 0,95 | 0,85 | 0,43 | 0,99 |
| | ≥ 10⁷ | 12 (7%) | 18 (90%) | 0,90 | 0,93 | 0,60 | 0,99 |
| | ≥ 10⁸ | 7 (4%) | 16 (80%) | 0,80 | 0,96 | 0,70 | 0,98 |
| | ≥ 10⁹ | 0 | 10 (50%) | 0,50 | 1,00 | 1,00 | 0,94 |
| *M*. *curtisii* | ≥ 10³ | 21 (12,6%) | 13 (65%) | 0,65 | 0,87 | 0,38 | 0,95 |
| | ≥ 10⁴ | 2 (1,2%) | 9 (45%) | 0,45 | 0,99 | 0,82 | 0,94 |
| | ≥ 10⁵ | 0 | 9 (45%) | 0,45 | 1,00 | 1,00 | 0,94 |
| | ≥ 10⁶ | 0 | 1 (5%) | 0,05 | 1,00 | 1,00 | 0,90 |
| *M. bominis* | ≥ 10³ | 22 (13%) | 10 (50%) | 0,50 | 0,87 | 0,31 | 0,94 |
| | ≥ 10⁴ | 13 (7,8%) | 7 (35%) | 0,35 | 0,92 | 0,35 | 0,92 |
| | ≥ 10⁵ | 6 (3,6%) | 7 (35%) | 0,35 | 0,96 | 0,54 | 0,93 |
| | ≥ 10⁶ | 2 (1,2%) | 6 (30%) | 0,30 | 0,99 | 0,75 | 0,92 |
| | ≥ 10⁷ | 2 (1,2%) | 6 (30%) | 0,30 | 0,99 | 0,75 | 0,92 |
| | ≥ 10⁸ | 2 (1,2%) | 1 (5%) | 0,05 | 0,99 | 0,33 | 0,90 |
| | ≥ 10⁹ | 1(0,6%) | 0 | 0 | 0,99 | 0 | 0,89 |
| *U. urealyticum* | ≥ 10³ | 22 (13%) | 5 (25%) | 0,25 | 0,87 | 0,18 | 0,91 |
| | ≥ 10⁴ | 17 (10%) | 5 (25%) | 0,25 | 0,90 | 0,23 | 0,91 |
| | ≥ 10⁵ | 13 (7,8%) | 3 (15%) | 0,15 | 0,92 | 0,19 | 0,90 |
| | ≥ 10⁶ | 7 (4,2%) | 0 | 0 | 0,96 | 0 | 0,89 |
| *Lactobacillus* | ≥ 10³ | 141 (84%) | 12 (60%) | 0,60 | 0,16 | 0,08 | 0,76 |
| spp. | ≥ 10⁴ | 134 (80%) | 7 (35%) | 0,35 | 0,20 | 0,05 | 0,72 |
| | ≥ 10⁵ | 116 (69%) | 4 (20%) | 0,20 | 0,30 | 0,03 | 0,76 |
| | ≥ 10⁶ | 106 (63%) | 4 (20%) | 0,20 | 0,36 | 0,04 | 0,79 |
| | ≥ 10⁷ | 97 (58%) | 3 (15%) | 0,15 | 0,42 | 0,03 | 0,80 |
| | ≥ 10⁸ | 74 (44%) | 0 | 0 | 0,55 | 0 | 1 |
| | ≥ 10⁹ | 13 (7,8%) | 0 | 0 | 0,92 | 0 | 1 |

| | | | | | | | |
|---|---|---|---|---|---|---|---|
| *Nbre de FN et nbre de VB identifiées sur 167 FN testées (%) et 20 VB testées (%)*Nbre de FN et nbre de VB identifiées sur 167 FN testées (%) et 20 VB testées (%) respectivement. **Sensibilité et Spécificité du seuil de quantification pour l'identification des VB ***VPP valeur prédictive positive et VPN valeur prédictive négative | | | | | | | |

**Tableau 5. Impact de l'association des critères de quantification par PCR en temps réel d'A. vaginae et G. vaginalis pour l'identification des vaginoses bactériennes. Le groupe de flores normales et des vaginoses bactériennes définies par le score de Nugent sont utilisés comme référence.**

| Micro-organismes associés | Seuil de quantifica-tion de *G. vaginalis* (bactéries/ml) | FN identifiées* | VB identifiées* | Sensibilité**^{¤} | Spéciticité** | VPP*** | VPN*** |
|---|---|---|---|---|---|---|---|
| *A. vaginae* ≥ 10⁷ et *G.* | ≥ 10⁶ | 5 (3%) | 18 (90%) | 0,90 | 0,97 | 0,78 | 0,99 |
| *vaginalis* | ≥ 10⁷ | 5(3%) | 17(85%) | 0,85 | 0,97 | 0,77 | 0,98 |
| | ≥ 10⁸ | 2(1,2%) | 15(75%) | 0,75 | 0,99 | 0,88 | 0,97 |
| | ≥ 10⁹ | 0 | 9(45%) | 0,45 | 1 | 1 | 0,94 |
| *A. vaginae* ≥ 10⁸ et *G.* | ≥ 10⁶ | 1(06%) | 17(85%) | 0,85 | 0,99 | 0,94 | 0,98 |
| *vaginalis* | ≥ 10⁷ | 1 (0,6%) | 17 (85%) | 0,85 | 0,99 | 0,94 | 0,98 |
| | ≥ 10⁸ | 0 | 15 (75%) | 0,75 | 1 | 1 | 0,97 |
| | ≥ 10⁹ | 0 | 9 (45%) | 0,45 | 1 | 1 | 0,94 |
| *A. vaginae* ≥ 10⁷ | ≥ 10⁶ | 26(15%) | 20 (100%) | 1 | 0,84 | 0,43 | 1 |
| et/ou *G. vaginalis* | ≥ 10⁷ | 13 (7,8%) | 20(100%) | 1 | 0,92 | 0,61 | 1 |
| | ≥ 10⁸ | 11 (6,6%) | 20 (100%) | 1 | 0,93 | 0,64 | 1 |
| | ≥ 10⁹ | 6 (3,6%) | 20 (100%) | 1 | 0,96 | 0,77 | 1 |
| *A. vaginae* ≥ 10⁸ | ≥ 10⁶ | 25(15%) | 20 (100%) | 1 | 0,85 | 0,44 | 1 |
| et/ou *G. vaginalis* | ≥ 10⁷ | 12(7,2%) | 19 (95%) | 0,95 | 0,93 | 0,61 | 0,99 |
| | ≥ 10⁸ | 7(4,2%) | 19(95%) | 0,95 | 0,96 | 0,73 | 0,99 |
| | ≥ 10⁹ | 1 | 19(95%) | 0,95 | 0,99 | 0,95 | 0,99 |

| | | | | | | | |
|---|---|---|---|---|---|---|---|
| *Nbre de FN et nbre de VB identifiées sur 167 FN testées (%) et 20 VB testées (%)*Nbre de FN et nbre de VB identifiées sur 167 FN testées (%) et 20 VB testées (%) respectivement. **Sensibilité et Spécificité du seuil de quantification pour l'identification des VB ***VPP valeur prédictive positive et VPN valeur prédictive négative | | | | | | | |

**Tableau 6. Suivis bactériologiques de 8 patientes présentant une vaginose bactérienne ou une flore intermédiaire par score de Nugent. Correspondance entre la classification par score de Nugent et l'identification de la VB par critères moléculaires.**

| **Sujet** | **Terme du prélèvement vaginal** | **Classification par Score de Nugent** | **Traitement** | **BV par critères moléculaires** | **Terme de l'accouchement** | **Poids nouveau né (gramme)** |
|---|---|---|---|---|---|---|
| **1** | 24 SA | VB | Oui | oui | 39 SA | 3420 |
| | 28 SA | FN | | | | |
| | 38 SA | FN | | oui | | |
| **2** | 27 SA | FI | | oui | 40 SA | 3090 |
| | 39 SA | FI | | oui | | |
| **3** | 6 SA | FN | | | 40 SA | 3490 |
| | 28 SA | FI | | | | |
| | 32 SA | FI | | | | |
| **4** | 6 SA | FI | | oui | 38 SA | 3580 |
| | 31 SA | FN | | | | |
| | 37 SA | FN | | | | |
| | 40 SA | FN | | | | |
| **5** | 26 SA | FI | | oui | 38 SA | 2970 |
| | 31 SA | FI | | oui | | |
| **6** | 31 SA | FN | | | 39 SA | 2700 |
| | 39 SA | FI | | oui | | |
| **7** | 25 SA | VB | Oui | oui | 37 SA | 4100 |
| | 29 SA | FN | | | | |
| **8** | 27 SA | VB | Oui | oui | 38 SA | 2700 |
| | 30 SA | FN | | | | |
| | 32 SA | FN | | | | |

## Revendications

1. Méthode de diagnostic et suivi in vitro de l'état de la flore bactérienne vaginale au regard de la présence d'une vaginose bactérienne, et le cas échéant pour le suivi de son traitement thérapeutique, **caractérisée en ce que**
- 1/ on quantifie les concentrations de bactéries Atopobium vaginae et Gardnerella vaginalis en
• déterminant les concentrations de séquences spécifiques desdites bactéries Atopobium vaginae et Gardnerella vaginalis présentes en une seule copie dans l'ADN desdites bactéries Atopobium vaginae et respectivement Gardnerella vaginalis, et d'une séquence spécifique d'un gène humain présent dans tout prélèvement biologique contenant des cellules humaines, dans l'ADN extrait d'un échantillon de sécrétion vaginale de patiente, lesdites séquences spécifiques présentant une taille inférieure à 150 nucléotides,
• par co-amplification enzymatique de type PCR desdites séquences spécifiques contenues, d'une part, dans ledit ADN extrait de l'échantillon et, d'autre part, dans des échantillons de fragments d'ADN synthétiques comprenant chacun desdites séquences spécifiques desdites bactéries et dite séquence spécifique d'un gène humain présent dans tout prélèvement biologique de cellules humaines, lesdits échantillons servant de standards d'étalonnage de quantification de l'ADN,
• les détection et quantification desdits fragments amplifiés étant réalisées à l'aide de sondes marquées de séquences distinctes de celles des amorces d'amplification pour chacune desdites séquences spécifiques desdites bactéries Atopobium vaginae et Gardnerella vaginalis et dite séquence spécifique d'un gène humain présent dans tout prélèvement biologique contenant des cellules humaines,
• lesdites séquences spécifiques desdites bactéries étant les séquences suivantes incluant des séquences desdites sondes (soulignées) encadrées par des séquences desdites amorces (en gras) ou leurs séquences complémentaires :
- pour *Atopobium vaginae:*
- pour *Gardnerella vaginalis:*
et
- 2/ on détermine la présence d'une vaginose bactérienne ou l'échec du traitement thérapeutique en cours, avec une valeur prédictive positive d'au moins 95% et une valeur prédictive négative d'au moins 99%, si les concentrations de fragments d'ADN des deux dites séquences spécifiques des bactéries Atopobium vaginae et respectivement Gardnerella vaginalis dans un échantillon de prélèvement de sécrétions vaginales de patiente contenant au moins 10⁴ cellules humaines/ml, sont telles que l'une au moins des 2 conditions a) et b) suivantes est respectée :
a) la concentration Ca en dit fragment d'ADN d'Atopobium vaginae est supérieure ou égale à 10⁸ copies/ml, et
b) la concentration Cg en dit fragment d'ADN de Gardnerella vaginalis est supérieure ou égale à 10⁹ copies/ml.

2. Méthode selon la revendication 1, **caractérisée en ce que** :
- a/ à l'étape 1/, on quantifie en outre les bactéries Lactobacillus sp. comprenant au moins les bactéries Lactobacillus acidophilus, Lactobacillus crispatus, Lactobacillus jensenii, Lactobacillus gasseri et Lactobacillus iners, en
• déterminant en outre la concentration d'une séquence spécifique desdites bactéries Lactobacillus sp., ladite séquence spécifique des Lactobacillus sp. étant présente en une seule copie dans l'ADN desdites bactéries Lactobacillus sp., et de taille inférieure à 150 nucléotides,
• par co-amplification enzymatique de type PCR additionnelle de ladite séquence spécifique des bactéries Lactobacillus sp. contenue, d'une part, dans ledit ADN extrait de l'échantillon et, d'autre part, dans un échantillon de fragments d'ADN synthétique comprenant, en outre, ladite séquence spécifique desdites bactérie Lactobacillus sp., ledit fragment d'ADN synthétique comprenant ladite séquence spécifique desdites bactéries Lactobacillus sp. servant de standard d'étalonnage de quantification,
• les détection et quantification desdits fragments amplifiés étant réalisées à l'aide de sondes marquées de séquences distinctes de celles des amorces d'amplification pour chacune desdites séquences spécifiques desdites bactéries Atopobium vaginae, Gardnerella vaginalis, Lactobacillus sp. et dite séquence spécifique d'un gène humain présent dans tout prélèvement biologique contenant des cellules humaines, et
- b/ à l'étape 2/, on détermine une vaginose bactérienne si, en outre, la concentration Cl en fragment d'ADN spécifique de dites bactéries Lactobacillus sp., est inférieure ou égale à 10⁸ copies/ml, de préférence inférieure ou égale à 10⁷ copies/ml.

3. Méthode selon la revendication 2, **caractérisée en ce qu'**on détermine une vaginose bactérienne si lesdites concentrations sont telles que les 3 conditions suivantes sont respectées :
a- concentration Ca en dit fragment d'ADN de séquence spécifique d'Atopobium vaginae supérieure ou égale à 10⁸ copies/ml,
b- concentration Cg en en dit fragment d'ADN de séquence spécifique de Gardnerella vaginalis supérieure ou égale à 10⁹ copies/ml, et
c- concentration (Cl) en dit fragment d'ADN de séquence spécifique de Lactobacillus sp. inférieure ou égale à 10⁷ copies/ml.

4. Méthode selon l'une des revendications 1 à 3, **caractérisée en ce que** l'on réalise des réactions d'amplification et quantification par PCR en Temps Réel, en mettant en oeuvre des sondes d'hydrolyse spécifiques respectivement de chacune desdites séquences spécifiques de dites bactéries et séquence spécifique d'un gène humain présent dans tout prélèvement biologique contenant des cellules humaines, dans l'échantillon à tester.

5. Méthode selon la revendication 4, **caractérisée en ce que** lesdites séquences spécifiques présentent une taille de 70 à 150 nucléotides, de préférence de 90 à 120 nucléotides.

6. Méthode selon l'une des revendications 1 à 5, **caractérisée en ce que** l'on met en oeuvre un grand fragment synthétique d'ADN servant de standard d'étalonnage de quantification de l'ADN, ledit grand fragment d'ADN synthétique regroupant desdites séquences spécifiques respectivement de chacune desdites bactéries dont les concentrations sont quantifiées, et ladite séquence d'ADN humain spécifique de cellules humaines.

7. Méthode selon la revendication 1 à 6, **caractérisée en ce que** lesdites séquences spécifiques desdites bactéries comprennent pour la bactérie Lactobacillus sp., une séquence commune aux bactéries Lactobacillus crispatus, Lactobacillus jensenii, Lactobacillus gasseri, Lactobacillus iners et Lactobacillus acidophilus au sein du gène tuf codant pour le facteur d'élongation aux positions 253 à 343.du gène de référence Genebank AY 562191.1.

8. Méthode selon la revendication 7, **caractérisée en ce que** ladite séquence spécifique pour Lactobacillus sp. est la séquence suivante incluant une séquence sonde (soulignée) encadrée par des séquences de dites amorces (en gras) ou leurs séquences complémentaires :

9. Méthode selon l'une des revendications 6 et 8, **caractérisée en ce que** ledit grand fragment d'ADN comprend, comme séquence spécifique d'ADN humain dans l'échantillon à tester, une séquence spécifique de l'albumine.

10. Méthode selon la revendication 9, **caractérisée en ce que** ladite séquence spécifique d'ADN humain dans l'échantillon à tester comprend le fragment des positions 16283-16423 de l'exon 12 du gène de l'albumine humaine de référence Genebank M12523.1 de séquence du listage de séquence suivante ou la séquence complémentaire:

11. Méthode selon la revendication 10, **caractérisée en ce que** ladite séquence spécifique d'ADN humain dans l'échantillon à tester comprend le fragment des positions 16283-16423 de l'exon 12 du gène de l'albumine humaine de référence Genebank M12523.1., est modifiée par insertion d'un site de clivage, notamment le site XhoI (séquence entre parenthèses) en dehors des séquences correspondant aux amorces (séquences en gras) et séquence sonde (séquence soulignée) de séquence du listage de séquence suivante ou la séquence complémentaire:

12. Méthode selon l'une des revendications 1 à 11, **caractérisée en ce que** l'on met en oeuvre les jeux d'amorces et de sondes choisis le cas échéant parmi les séquences suivantes du listage de séquence ou respectivement leurs séquences complémentaires:
- pour Atopobium vaginae:
Amorce 5' : Seq. n°5 = 5'-CCCTATCCGCTCCTGATACC-3'
Amorce 3' :Seq. n°6= 5'-CCAAATATCTGCGCATTTCA-3'
Sonde: Seq. n°7 =5'-GCAGGCTTGAGTCTGGTAGGGGA-3'
- pour Gardnerella vaginalis:
Amorce 5' : Seq. n°8 = 5'-CGCATCTGCTAAGGATGTTG-3'
Amorce 3' : Seq. n°9= 5'-CAGCAATCTTTTCGCCAACT-3'
Sonde: Seq. n°10= 5'-TGCAACTATTTCTGCAGCAGATCC-3'
- pour Lactobacillus sp. :
Amorce 5' :Seq. n°11= 5'-TACATCCCAACTCCAGAACG-3'
Amorce 3' : Seq. n°12 = 5'-AAGCAACAGTACCACGACCA-3'
Sonde:Seq. n°13 = 5'-TGACAAGCCATTCTTAATGCA-3',
- pour l'albumine humaine :
Amorce 5' : Seq. n°14 = 5'-GCTGTCATCTCTTGTGGGCTGT-3'
Amorce 3' : Seq. n°15 = 3-'AAACTCATGGGAGCTGCTGGTTC-3'
Sonde:Seq. n°16 = 5'-CCTGTCATGCCCACACAAATCTCTCC-3'

13. Méthode selon l'une des revendications 6 à 12, **caractérisée en ce que** ledit grand fragment d'ADN synthétique constitutif de l'ADN de l'échantillon standard d'étalonnage de la quantification est inséré dans un plasmide.

14. Trousse de diagnostic utile pour la mise en oeuvre d'une méthode selon l'une des revendications 1 à 13, **caractérisée en ce qu'**elle comprend
- des échantillons d'ADN standard d'étalonnage comprenant desdites séquences spécifiques de chacun desdites bactéries telles que définies dans l'une des revendications 1, 5, 7 et 8, de préférence une dite séquence spécifique de l'ADN humain telle que définie dans les revendications 1, 9, 10 et 11, et, de préférence encore, un dit grand fragment d'ADN synthétique tel que défini dans les revendications 6 et 13, ainsi que
- desdits jeux d'amorces spécifiques desdits fragments d'ADN synthétiques modifiés spécifiques desdites bactéries et de préférence encore, desdites sondes tels que définis dans l'une des revendications 1, 4, 8 et 12, et
- des réactifs de mise en oeuvre d'une réaction d'amplification d'ADN de type PCR.

## Claims

1. Method for *in vitro* diagnosis and follow-up of the vaginal bacterial flora status in relation to the presence of bacterial vaginosis, and for monitoring its treatment where applicable, **characterized in that**:
- 1) the concentrations of the bacteria *Atopobium vaginale* and *Gardnerella vaginalis* are quantified by
• determining the concentrations of the specific sequences of said bacteria *Atopobium vaginale* and *Gardnerella vaginalis* present in a single copy in the DNA of said bacteria *Atopobium vaginale* and *Gardnerella vaginalis,* and of a specific sequence of a human gene present in all biological specimens containing human cells, in the DNA extracted from a vaginal discharge specimen from a patient, said specific sequences being less than 150 nucleotides in size,
• enzymatic co-amplification of the PCR type of said specific sequences contained, on the one hand, in said DNA extracted from the specimen and, on the other hand, in samples of synthetic DNA fragments including each of said specific sequences of said bacteria and said specific sequence of a human gene present in all biological human cell specimens, said samples serving as calibration standards for quantifying the DNA,
• detection and quantification of said amplified fragments being carried out with the aid of probes labeled with sequences different from those of the amplification primers for each of said specific sequences of said bacteria *Atopobium vagiuae* and *Gardnerella vaginalis* and said specific sequence of a human gene present in all biological human cell specimens,
• said specific sequences of said bacteria being the following sequences, including probe sequences (underlined) framed by primer sequences (boldfaced) or their complementary sequences :
- for *Atopobium vaginae*:
- for *Gardnerella vaginalis*:
and
- 2) determining the presence of bacterial vaginosis or failure of the on-going treatment if the concentrations of DNA fragments of said two specific sequences of the bacteria *Atopobium vaginale* and *Gardnerella vaginalis* in a specimen of the patient's vaginal discharge containing at least 10⁴ human cells/mL are such that at least one of the following two conditions a) and b) is met:
a) the concentration Ca of said DNA fragment of *Atopobium vaginale* is greater than or equal to 10⁸ copies/mL, and
b) the concentration Cg of said DNA fragment of *Gardnerella vaginalis* is greater than or equal to 10⁹ copies/mL.

2. Method according to Claim 1, **characterized in that**:
- a) in step 1), the *Lactobacillus* sp. including at least the bacteria *Lactobacillus acidophilus*, *Lactobacillus crispatus*, *Lactobacillus jenseneii, Lactobacillus gasseri* and *Lactobacillus iners* are additionally quantified
• by additionally determining the concentration of a specific sequence of said *Lactobacillus* sp. bacteria, said specific sequence of *Lactobacillus* sp. being present in a single copy in the DNA of said *Lactobacillus* sp. bacteria, and being less than 150 nucleotides in size,
• by additional enzymatic co-amplification of the PCR type of said specific sequence of *Lactobacillus* sp. contained, on the one hand, in said DNA extracted from the specimen and, on the other hand, in a specimen of synthetic DNA fragments including, additionally, said specific sequence of said *Lactobacillus* sp. bacteria, with said synthetic DNA fragment including said specific sequence of said *Lactobacillus* sp. bacteria serving as a quantification reference standard.
• detection and quantification of said amplified fragments being accomplished with the aid of labeled probes with sequences different from those of the amplification primers for each of said specific sequences of said *Atopobium vaginae, Gardnerella vaginalis,* and *Lactobacillus* sp. bacteria and said specific sequence of a human gene present in any biological specimen containing human cells, and
- b) in step 2), bacterial vaginosis is determined if, additionally, the Cl concentration of a specific DNA fragment of said *Lactobacillus* sp. bacteria is less than or equal to 10⁸ copies/mL, preferably less than or equal to 10⁷ copies/mL.

3. Method according to Claim 2, **characterized in that** bacterial vaginosis is determined if said concentrations are such that the following three conditions are met:
a- Concentration Ca of said DNA fragment of a specific sequence of *Atopobium vaginae* is greater than or equal to 10⁸ copies/mL,
b- Concentration Cg of said DNA fragment of a specific sequence of *Gardnerella vaginalis* is greater than or equal to 10⁹ copies/mL, and
c- Concentration Cl of said DNA fragment of a specific sequence of *Lactobacillus* sp. is less than or equal to 10⁷ copies/mL.

4. Method according to one of Claims 1 to 3, **characterized in that** real-time PCR amplification and quantification reactions are performed by using hydrolysis probes specific to each of said specific sequences of said bacteria and specific sequence of a human gene present in any biological specimen containing human cells, in the specimen to be tested.

5. Method according to Claim 4, **characterized in that** said specific sequences are 70 to 150 nucleotides in size, preferably 90 to 120 nucleotides.

6. Method according to one of Claims 1 to 5, **characterized in that** a large synthetic DNA fragment serving as a reference standard for DNA quantification is used, said large synthetic DNA fragment grouping said specific sequences of each of said bacteria whose concentrations are quantified, and said specific human DNA sequence of human cells.

7. Method according to Claims 1 to 6, **characterized in that** said specific sequences of said bacteria include for the *Lactobacillus* sp. bacterium, a sequence common to the bacteria *Lactobacillus crispatus, Lactobacillus jenseneii, Lactobacillus gasseri, Lactobacillus iners* and *Lactobacillus acidophilus* in the *tuf* gene coding for the elongation factor at positions 253 to 343 of the gene with GenBank reference AY 562191.1.

8. Method according to Claim 7, **characterized in that** said specific sequence for *Lactobacillus* sp. is the following sequence, including a probe sequence (underlined) framed by primer sequences (boldfaced) or their complementary sequences:

9. Method according to one of Claims 6 and 8, **characterized in that** said large DNA fragment includes, as a specific sequence of human DNA in the test sample, a specific albumin sequence.

10. Method according to Claim 9, **characterized in that** said specific sequence of human DNA in the test specimen includes the fragment of positions 16283-16423 of exon 12 of the human albumin gene with GenBank reference M12523.1 with the following sequence or complementary sequence listing:

11. Method according to Claim 10, **characterized in that** said specific sequence of the human DNA in the test specimen including the fragment of positions 16283-16423 of exon 12 of the human albumin gene GenBank reference M12523.1, is modified by insertion of a cleavage site, particularly site XhoI, outside the sequences corresponding to the primers (boldfaced sequences) and the probe sequence (underlined sequence) of the following sequence listing:

12. Method according to one of Claims 1 to 11, **characterized by** using sets of primers and probes, chosen where applicable from the following sequences of the sequence listing or their complementary sequences:
- for *Atopobium vaginae:*
Primer 5' : Seq. No. 5 = 5'-CCCTATCCGCTCCTGATACC-3'
Primer 3' : Seq. No. 6 = 5'-CCAAATATCTGCGCATTTCA-3' Probe : Seq. No. 7 = 5'-GCAGGCTTGAGTCTGGTAGGGGA-3'
- for *Gardnerella vaginalis:*
Primer 5' : Seq. No. 8 = 5'-CGCATCTGCTAAGGATGTTG-3'
Primer 3' : Seq. No. 9 = 5'-CAGCAATCTTTTCGCCAACT-3'
Probe : Seq. No. 10 = 5'-TGCAACTATTTCTGCAGCAGATCC-3'
- for *Lactobacillus* sp.:
Primer 5' :Seq. No. 11 = 5'-TACATCCCAACTCCAGAACG-3'
Primer 3' : Seq. No. 12 = 5'-AAGCAACAGTACCCACGACCA-3'
Probe : Seq. No. 13 = 5'-TGACAAGCCATTCTTAATGCA-3',
- for human albumin:
Primer 5' : Seq. No. 14 = 5'-GCTGTCATCTCTTGTGGGCTGT-3'
Primer 3' : Seq. No. 15 = 3'-AAACTCATGGGAGCTGCTGGTTC-3'
Probe :Seq. No. 16 = 5'-CCTGTCATGCCCACACAAATCTCTCC-3'

13. Method according to one of Claims 6 to 12, **characterized in that** said large synthetic DNA fragment constituting the DNA of the standard reference specimen for quantification is inserted into a plasmid.

14. Diagnostic kit used for implementing a method according to one of Claims 1 to 13, **characterized by** including:
- samples of standard reference DNA including said specific sequences of each of said bacteria as defined in one of Claims 1, 5, 7, and 8, preferably one said specific sequence of human DNA as defined in Claims 1, 9, 10, and 11, and more preferably one said large fragment of synthetic DNA as defined in Claims 6 and 13, as well as
- said sets of specific primers of said modified synthetic DNA fragments specific to said bacteria, and more preferably, said probes as defined in one of Claims 1, 4, 8, and 12, and
- reagents for implementing a PCR-type DNA amplification reaction.

## Patentansprüche

1. Verfahren zur In-vitro-Diagnostik und -Beobachtung des Zustandes der bakteriellen Vaginalflora in Hinblick auf das Vorliegen einer bakteriellen Vaginose sowie gegebenenfalls zur Beobachtung ihrer therapeutischen Behandlung, **dadurch gekennzeichnet, daß**
- 1/ die Konzentrationen von Atopobium vaginae- und Gardnerella vaginalis-Bakterien quantifiziert werden durch
- Bestimmen der Konzentrationen spezifischer Sequenzen der Bakterien Atopobium vaginae und Gardnerella vaginalis, die in einer einzigen Kopie in der DNA der Bakterien Atopobium vaginae bzw. Gardnerella vaginalis vorhanden sind, sowie einer spezifischen Sequenz eines menschlichen Gens, das in jeder menschliche Zellen enthaltenden biologischen Probe vorhanden ist, in der aus einer Vaginalsekretprobe einer Patientin extrahierten DNA, wobei die spezifischen Sequenzen eine Größe von weniger als 150 Nukleotiden aufweisen,
- durch enzymatische Co-Amplifikation vom Typ PCR der spezifischen Sequenzen, die einerseits in der aus der Probe extrahierten DNA und andererseits in Proben von synthetischen DNA-Fragmenten enthalten sind, die jeweils spezifische Sequenzen der Bakterien und eine spezifische Sequenz eines menschlichen Gens, das in jeder biologischen Probe von menschlichen Zellen vorhanden ist, umfassen, wobei die Proben als Kalibrierungsstandards zur Quantifizierung der DNA dienen,
- wobei die Detektion und die Quantifizierung der amplifizierten Fragmente mit Hilfe von Sonden, die mit anderen Sequenzen als die Amplifikationsprimer markiert sind, für jede der spezifischen Sequenzen der Bakterien Atopobium vaginae und Gardnerella vaginalis und die spezifische Sequenz eines menschlichen Gens, das in jeder menschliche Zellen enthaltenden biologischen Probe vorhanden ist, durchgeführt werden,
- wobei die spezifischen Sequenzen der Bakterien die folgenden Sequenzen sind, welche Sequenzen der Sonden (unterstrichen), eingerahmt von Sequenzen der Primer (fettgedruckt), oder deren komplementäre Sequenzen einschließen:
- für Atopobium vaginae:
- für Gardnerella vaginalis:
und
- 2/ das Vorliegen einer bakteriellen Vaginose oder der Mißerfolg der laufenden therapeutischen Behandlung, mit einem positiven prädiktiven Wert von wenigstens 95 % und einem negativen prädiktiven Wert von wenigstens 99 %, bestimmt wird, wenn die Konzentrationen von DNA-Fragmenten der beiden spezifischen Sequenzen der Bakterien Atopobium vaginae bzw. Gardnerella vaginalis in einer Probe von entnommenen Vaginalsekreten einer Patientin, die wenigstens 10⁴ menschliche Zelle/ml enthält, derart sind, das wenigstens eine der beiden folgenden Bedingungen a) und b) eingehalten wird:
a) die Ca-Konzentration an DNA-Fragment von Atopobium vaginae ist größer oder gleich 10⁸ Kopien/ml und
b) die Cg-Konzentration an DNA-Fragment von Gardnerella vaginalis ist größer oder gleich 10⁹ Kopien/ml.

2. Verfahren nach Anspruch 1, **dadurch gekennzeichnet, daß**:
- a/ bei Schritt 1/ ferner die Bakterien Lactobacillus sp., wenigstens umfassend die Bakterien Lactobacillus acidophilus, Lactobacillus crispatus, Lactobacillus jensenii, Lactobacillus gasseri und Lactobacillus iners, **dadurch** quantifiziert werden, daß
- ferner die Konzentration einer spezifischen Sequenz der Bakterien Lactobacillus sp. bestimmt wird, wobei die spezifische Sequenz der Lactobacillus sp. in einer einzigen Kopie in der DNA der Bakterien Lactobacillus sp. vorliegt und eine Größe von weniger als 150 Nukleotiden aufweist,
- durch zusätzliche enzymatische Co-Amplifikation vom Typ PCR der spezifischen Sequenz der Bakterien Lactobacillus sp., die einerseits in der aus der Probe extrahierten DNA und andererseits in einer Probe synthetischer DNA-Fragmente enthalten ist, die ferner die spezifische Sequenz der Bakterien Lactobacillus sp. umfaßt, wobei das synthetische DNA-Fragment, welches die spezifische Sequenz der Bakterien Lactobacillus sp. umfaßt, als Kalibrierungsstandard der Quantifizierung dient,
- wobei die Detektion und die Quantifizierung der amplifizierten Fragmente mit Hilfe von Sonden, die mit anderen Sequenzen als die Amplifikationsprimer markiert sind, für jede der spezifischen Sequenzen der Bakterien Atopobium vaginae, Gardnerella vaginalis, Lactobacillus sp. und die spezifische Sequenz eines menschlichen Gens, das in jeder menschliche Zellen enthaltenden biologischen Probe vorhanden ist, durchgeführt werden, und
- b/ bei Schritt 2/ eine bakterielle Vaginose bestimmt wird, wenn außerdem die Cl-Konzentration an spezifischem DNA-Fragment von Lactobacillus sp.-Bakterien kleiner oder gleich 10⁸ Kopien/ml, vorzugsweise kleiner oder gleich 10⁷ Kopien/ml ist.

3. Verfahren nach Anspruch 2, **dadurch gekennzeichnet, daß** eine bakterielle Vaginose bestimmt wird, wenn die Konzentrationen derart sind, daß die drei folgenden Bedingungen eingehalten werden:
a- Ca-Konzentration an DNA-Fragment einer spezifischen Sequenz von Atopobium vaginae größer oder gleich 10⁸ Kopien/ml,
b- Cg-Konzentration an DNA-Fragment einer spezifischen Sequenz von Gardnerella vaginalis größer oder gleich 10⁹ Kopien/ml und
c- (Cl)-Konzentration an DNA-Fragment einer spezifischen Sequenz von Lactobacillus sp. kleiner oder gleich 10⁷ Kopien/ml.

4. Verfahren nach einem der Ansprüche 1 bis 3, **dadurch gekennzeichnet, daß** Amplifikations- und Quantifizierungsreaktionen mittels Echtzeit-PCR unter Einsatz von spezifischen Hydrolyse-Sonden einer jeden der spezifischen Sequenzen von Bakterien und einer spezifischen Sequenz eines menschlichen Gens, das in jeder menschliche Zellen enthaltenden biologischen Probe vorhanden ist, in der zu untersuchenden Probe durchgeführt werden.

5. Verfahren nach Anspruch 4, **dadurch gekennzeichnet, daß** die spezifischen Sequenzen eine Größe von 70 bis 150 Nukleotiden, vorzugsweise von 90 bis 120 Nukleotiden aufweisen.

6. Verfahren nach einem der Ansprüche 1 bis 5, **dadurch gekennzeichnet, daß** ein großes synthetisches DNA-Fragment eingesetzt wird, das als Kalibrierungsstandard zur Quantifizierung der DNA dient, wobei das große synthetische DNA-Fragment spezifische Sequenzen jeweils eines jeden der Bakterien, deren Konzentrationen quantifiziert werden, sowie die spezifische menschliche DNA-Sequenz menschlicher Zellen zusammenfaßt.

7. Verfahren nach Anspruch 1 bis 6, **dadurch gekennzeichnet, daß** die spezifischen Sequenzen der Bakterien für das Bakterium Lactobacillus sp. eine den Bakterien Lactobacillus crispatus, Lactobacillus jensenii, Lactobacillus gasseri, Lactobacillus iners und Lactobacillus acidophilus gemeinsame Sequenz innerhalb des tuf-Gens, das für den Elongationsfaktor an den Positionen 253 bis 343 des Gens mit der Referenz GenBank AY 562191.1 kodiert, umfassen.

8. Verfahren nach Anspruch 7, **dadurch gekennzeichnet, daß** die spezifische Sequenz für Lactobacillus sp. die folgende Sequenz ist, welche eine Sonden-Sequenz (unterstrichen), eingerahmt von Sequenzen von Primern (fettgedruckt), oder deren komplementäre Sequenzen einschließt:

9. Verfahren nach einem der Ansprüche 6 und 8, **dadurch gekennzeichnet, daß** das große DNA-Fragment als spezifische Sequenz menschlicher DNA in der zu untersuchenden Probe eine spezifische Sequenz des Albumin umfaßt.

10. Verfahren nach Anspruch 9, **dadurch gekennzeichnet, daß** die spezifische Sequenz menschlicher DNA in der zu untersuchenden Probe das Fragment der Positionen 16283-16423 des Exon 12 des Gens des Humanalbumin mit der Referenz GenBank M12523.1 umfaßt, mit folgender Sequenz des Sequenzprotokolls oder der komplementären Sequenz:

11. Verfahren nach Anspruch 10, **dadurch gekennzeichnet, daß** die spezifische Sequenz menschlicher DNA in der zu untersuchenden Probe das Fragment der Positionen 16283-16423 des Exon 12 des Gens des Humanalbumin mit der Referenz GenBank M12523.1 umfaßt, durch Einfügen einer Schnittstelle, insbesondere der Xhol-Stelle (Sequenz in Klammern) außerhalb der Sequenzen, die den Primern (fettgedruckte Sequenzen) entsprechen, und der Sonden-Sequenz (unterstrichene Sequenz) modifiziert ist, mit folgender Sequenz des Sequenzprotokolls oder der komplementären Sequenz:

12. Verfahren nach einem der Ansprüche 1 bis 11, **dadurch gekennzeichnet, daß** die Sätze von Primern und Sonden, die gegebenenfalls aus den folgenden Sequenzen des Sequenzprotokolls oder jeweils ihren komplementären Sequenzen ausgewählt sind, verwendet werden:
- für Atopobium vaginae:
Primer 5': Seq. N°5 = 5'-CCCTATCCGCTCCTGATACC-3'
Primer 3': Seq. N°6 = 5'-CCAAATATCTGCGCATTTCA-3'
Sonde: Seq. N°7 = 5'-GCAGGCTTGAGTCTGGTAGGGGA-3'
- für Gardnerella vaginalis:
Primer 5': Seq. N°8 = 5'-CGCATCTGCTAAGGATGTTG-3'
Primer 3': Seq. N°9 = 5'-CAGCAATCTTTTCGCCAACT-3'
Sonde: Seq. N°10 = 5'-TGCAACTATTTCTGCAGCAGATCC-3'
- für Lactobacillus sp.:
Primer 5': Seq. N°11 = 5'-TACATCCCAACTCCAGAACG-3'
Primer 3': Seq. N°12 = 5'-AAGCAACAGTACCACGACCA-3'
Sonde: Seq. N°13 = 5'-TGACAAGCCATTCTTAATGCA-3'
- für Humanalbumin:
Primer 5': Seq. N°14 = 5'-GCTGTCATCTCTTGTGGGCTGT-3'
Primer 3': Seq. N°15 = 3'-AAACTCATGGGAGCTGCTGGTTC-3'
Sonde: Seq. N°16 = 5'-CCTGTCATGCCCACACAAATCTCTCC-3'

13. Verfahren nach einem der Ansprüche 6 bis 12, **dadurch gekennzeichnet, daß** das große synthetische DNA-Fragment, welches Bestandteil der DNA der Standardprobe zur Kalibrierung der Quantifizierung ist, in ein Plasmid eingesetzt wird.

14. Diagnose-Kit für die Durchführung eines Verfahrens nach einem der Ansprüche 1 bis 13, **dadurch gekennzeichnet, daß** es umfaßt:
- Standard-DNA-Proben zur Kalibrierung, umfassend spezifische Sequenzen eines jeden der Bakterien wie sie in einem der Ansprüche 1, 5, 7 und 8 definiert sind, vorzugsweise eine spezifische Sequenz der menschlichen DNA wie sie in den Ansprüchen 1, 9, 10 und 11 definiert ist, und weiterhin vorzugsweise ein großes synthetisches DNA-Fragment, wie es in den Ansprüchen 6 und 13 definiert ist, sowie
- Sätze von spezifischen Primern der modifizierten, spezifischen synthetischen DNA-Fragmente der Bakterien und weiterhin vorzugsweise Sonden wie sie in einem der Ansprüche 1, 4, 8 und 12 definiert sind, sowie
- Reagenzien zur Durchführung einer DNA-Amplifikationsreaktion vom Typ PCR.
